# EUROPEAN PATENT APPLICATION

(11) **EP 1 270 566 A1**
(43) Date of publication of application: **02.01.2003**
(21) Application number: 01921792.6
(22) Date of filing: 04.04.2001
(51) Int. Cl.: C07D 331/02, C08G 75/06, G02B 1/04

(54) **EPISULFIDE COMPOUND AND PROCESS FOR PRODUCING THE SAME**

(30) Priority: 07.04.2000 JP 2000106867
(71) Applicant: MITSUBISHI GAS CHEMICAL COMPANY, INC., Tokyo 100-0005 (JP)
(72) Inventor: Amagai, A. Mitsubishi Gas Chemical Company, Inc., Tokyo 125-0051 (JP); Yoshimura, Y. Mitsubishi Gas Chemical Company, Inc, Tokyo 125-005 (JP); Takeuchi, M. Mitsubishi Gas Chemical Company, Inc., Tokyo 125-005 (JP); Niimi, A. Mitsubishi Gas Chemical Company, Inc., Tokyo 125-0051 (JP); Horikoshi, H. Mitsubishi Gas Chemical Company, Inc, Tokyo 125-005 (JP); Shimuta, M. Mitsubishi Gas Chemical Company, Inc., Osaka-shi, Osaka 551-0022 (JP); Uemura, N. Mitsubishi Gas Chemical Company, Inc., Osaka-shi, Osaka-shi, Osaka 551-0022 (JP)
(74) Representative: Gille Hrabal Struck Neidlein Prop Roos
(86) International application number: JP0102927
(87) International publication number: WO01077098

(57) **Abstract**

The episulfide compound of the present invention has, in one molecule, one or more epithio structures represented by the following Formula 1: 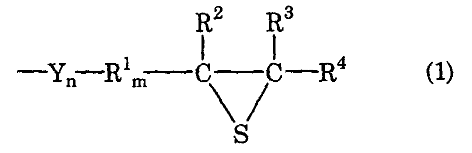 wherein R¹ is C₁-C₁₀ hydrocarbylene group; R², R³ and R⁴ are each independently C₁-C₁₀ hydrocarbyl group or hydrogen; Y is S, O, Se or Te; n is an integer of from 0 to 5; and m is 0 or 1, and further characterized by having a nitrogen content of 5000 ppm or less. The optical material produced by curing the episulfide compound by polymerization has a high refractive index and a large Abbe's number with little coloring and haze. The optical material is useful, particularly, as spectacle plastic lenses.

## Description

### Technical Field

The present invention relates to a production method of a monomer compound suitable for producing an optical material such as plastic lens, prism, optical fiber, information recording medium, and filter, particularly, for producing a plastic spectacle lens.

### Background Art

Plastic materials have been widely used in various optical applications, particularly in manufacturing spectacle lenses, because of their light weight, toughness and easiness of dyeing. Optical products, particularly spectacle lenses are required to have, in addition to a low specific gravity, optical properties such as a high refractive index and a large Abbe's number and physical properties such as high heat resistance and large mechanical strength. A high refractive index can decrease thickness of a lens. A large Abbe's number is important to avoid chromatic aberration of a lens. A high heat resistance and a large mechanical strength are important to facilitate fabrication and also for safety.

As high refractive index materials, thermosetting optical materials having a thiourethane structure derived from the reaction of a polythiol compound and a polyisocyanate compound have been proposed in Japanese Patent Publication Nos. 4-58489 and Japanese Patent Application Laid-Open No. 5-148340. Japanese Patent Application Laid-Open Nos. 1-98615 and 3-81320 and International Publication WO/89 10575 disclose the production of lenses by polymerization of an epoxy resin or an episulfide resin with a multifunctional compound. However, it has been still demanded to increase the refractive index without increasing the chromatic aberration. In other words, a material simultaneously satisfying high refractive index and large Abbe's number has been demanded.

To meet the demand, the inventors found novel sulfur-containing episulfide compounds which were capable of providing thin, low chromatic aberration optical materials having a refractive index of 1.7 or more and an Abbe's number of 35 or more (Japanese Patent Application Laid-Open Nos. 9-71580, 9-110979 and 9-255781). To produce the proposed episulfide compounds by the reaction of a corresponding epoxy compound with thiourea, it is advantageous to proceed the reaction in a mixed solvent of water or a water-soluble solvent such as alcohols and a water-insoluble solvent such as aromatic or halogenated hydrocarbons because both of thiourea and the episulfide compound dissolve in such a mixed solvent. However, in this reaction, unreacted thiourea, by-produced urea and nitrogen compound, etc. remain in the reaction liquid. A lens produced from an episulfide compound contaminated with these compounds has problems such as coloring and haze. Thus, the above production method fails to provide an episulfide compound capable of producing a lens with satisfactory performance.

### Disclosure of Invention

An object of the present invention is to provide an episulfide compound capable of producing a high refractive index, high Abbe's number optical material with little coloring and haze.

After extensive study in view of the above object, the inventors have found that the content of nitrogen attributable to thiourea, the by-produced urea, etc. in an episulfide compound can be reduced to 5000 ppm or less by reacting thiourea with an epoxy compound having, in one molecule, one or more epoxy structures per one molecule represented by the following Formula 2: 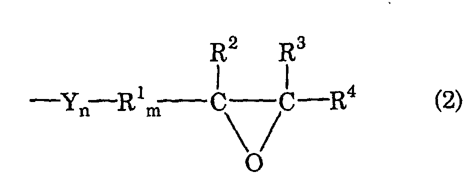 wherein R¹ is C₁-C₁₀ hydrocarbylene group; R², R³ and R⁴ are each independently C₁-C₁₀ hydrocarbyl group or hydrogen; Y is S, O, Se or Te; n is an integer of from 0 to 5; and m is 0 or 1, in a mixed solvent of a water-soluble solvent and a water-insoluble solvent, adding the water-soluble solvent and/or the water-insoluble solvent to the resultant reaction liquid; mixing the resultant mixture and separating the mixture into an aqueous layer and a non-aqueous layer; and isolating from the non-aqueous layer an episulfide compound having, in one molecule, one or more epithio structures represented by the following Formula 1: 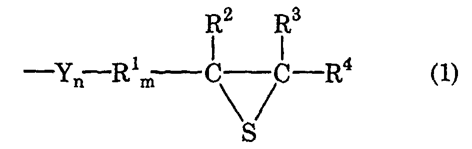 wherein R¹, R², R³, R⁴, Y, m and n are as defined above. The inventors have further found that the curing by polymerization of the episulfide compound thus obtained provides a lens with less coloring and haze. The invention has been accomplished based on these findings.

Thus, in a first aspect of the present invention, there is provided an episulfide compound having, in one molecule, one or more epithio structures represented by the Formula 1, and having a nitrogen content of 5000 ppm or less.

In a second aspect of the present invention, there is provided a method for producing an episulfide compound having a nitrogen content of 5000 ppm or less, the method comprising the steps of reacting thiourea with an epoxy compound having, in one molecule, one or more epoxy structures represented by the Formula 2 in a mixed solvent of a water-soluble solvent and a water-insoluble solvent; adding the water-soluble solvent and/or the water-insoluble solvent to the resultant reaction liquid; mixing the resultant mixture and separating the mixture into an aqueous layer and a non-aqueous layer; and isolating from the non-aqueous layer the episulfide compound. The nitrogen content of the episulfide compound is reduced to 5000 ppm or less by the washing treatment of the reaction product, comprising the addition of a water-insoluble solvent and/or a water-soluble solvent which may contain an acid, and the subsequent mixing and separation.

### Best Mode for Carrying Out the Invention

In the present invention, the episulfide compound is produced from an epoxy compound by using thiourea. Thiourea is used stoichiometrically in an equimolar amount to the epoxy groups of the epoxy compound. A smaller or higher amount is applicable when taking much account of the purity of the product, the reaction rate, the production cost, etc. The use amount of thiourea is preferably from 1 to 5 times the stoichiometric amount, more preferably from 1 to 2.5 times the stoichiometric amount. The reaction temperature is -20 to 100°C, preferably 0 to 70°C. The reaction time is not particularly limited so long as the reaction is completed under the above conditions. The reaction is preferably conducted with stirring to promote the reaction. The reaction is also preferably conducted in an inert gas atmosphere such as nitrogen because the side reaction is prevented. The addition of an acid or an acid anhydride to the reaction system as a polymerization inhibitor is effective in view of increasing the yield of reaction. Examples of the acid and acid anhydride include nitric acid, hydrochloric acid, sulfuric acid, fuming sulfuric acid, boric acid, arsenic acid, phosphoric acid, prussic acid, acetic acid, peracetic acid, thioacetic acid, oxalic acid, tartaric acid, propionic acid, butyric acid, succinic acid, maleic acid, benzoic acid, anhydrous nitric acid, anhydrous sulfuric acid, boron oxide, arsenic pentoxide, phosphorus pentoxide, anhydrous chromic acid, acetic anhydride, propionic anhydride, butyric anhydride, succinic anhydride, maleic anhydride, benzoic anhydride, phthalic anhydride, silica gel, silica-alumina, and aluminum chloride. These compounds may be used in combination. The addition amount is usually 0.001 to 10 parts by weight, preferably 0.01 to 1 part by weight per 100 parts by weight of the reaction liquid.

In the present invention, the water-insoluble solvent for the mixed reaction solvent means a solvent showing substantially no or extremely low solubility to water. Examples thereof include ethers such as diethyl ether; aliphatic hydrocarbons such as hexane, heptane and cyclohexane; aromatic hydrocarbons such as benzene, toluene and xylene; and halogenated hydrocarbons such as dichloromethane, dichloroethane, chloroform and chlorobenzene, with the aromatic hydrocarbon such as toluene and the halogenated hydrocarbons such as dichloromethane being preferred. The water-soluble solvent, the other solvent for the mixed reaction solvent, means a solvent showing a substantial solubility to water. Examples thereof include water; alcohols such as methanol, ethanol and isopropyl alcohol; ethers such as tetrahydrofuran and dioxane; hydroxyethers such as methyl cellosolve, ethyl cellosolve and butyl cellosolve; ketones such as acetone; and polar solvents such as dimethylformamide, dimethylacetamide and N-methylpyrrolidone, with water and the alcohols such as methanol, ethanol and isopropyl alcohol being preferred.

The reaction of the present invention is carried out in a water-soluble solvent/water-insoluble solvent (10/1 to 1/10 by weight) mixture at an epoxy compound concentration of 0.001 to 20% by weight using thiourea in an amount of 1 to 5 times the stoichiometric amount. The reaction liquid separates into an aqueous layer containing thiourea and by-produced urea and a non-aqueous layer containing the episulfide compound or, as the case may be, the reaction liquid does not separate into the layers.

The reaction liquid is separated into the aqueous layer and the non-aqueous layer by adding a water-insoluble solvent and/or a water-soluble solvent and mixing, and then, the aqueous layer is discarded.

The water-insoluble solvent to be added to the reaction liquid means a solvent showing substantially no or extremely low solubility to water. Examples thereof include ethers such as diethyl ether; aliphatic hydrocarbons such as hexane, heptane and cyclohexane; aromatic hydrocarbons such as benzene, toluene and xylene; and halogenated hydrocarbons such as dichloromethane, dichloroethane, chloroform and chlorobenzene. Although the water-insoluble solvent to be added to the reaction liquid may be the same as or different from the water-insoluble solvent used as the reaction solvent, the solvents are preferred to be the same because the solvents can be easily recovered. The addition amount is usually 0.1 to 200 parts by weight, preferably 0.1 to 100 parts by weight, and more preferably 0.1 to 50 parts by weight per 100 parts by weight of the reaction liquid.

The water-soluble solvent to be added to the reaction liquid means a solvent showing a substantial solubility to water. Examples thereof include water; alcohols such as methanol, ethanol and isopropyl alcohol; ethers such as tetrahydrofuran and dioxane; hydroxyethers such as methyl cellosolve, ethyl cellosolve and butyl cellosolve; ketones such as acetone; and polar solvents such as dimethylformamide, dimethylacetamide and N-methylpyrrolidone. The water-soluble solvent may contain an acid. Water and an aqueous solution of acid are preferably, and the aqueous solution of acid is particularly preferably used as the water-soluble solvent. The addition amount is usually 0.1 to 200 parts by weight, preferably 0.1 to 100 parts by weight, and more preferably 0.1 to 50 parts by weight per 100 parts by weight of the reaction liquid.

Either of the water-insoluble solvent and the water-soluble solvent may be added to the reaction liquid, preferably both solvents, more preferably the water-insoluble solvent and the aqueous solution of acid are added. Either solvent may be added first, or both solvents may be added simultaneously. For example, the water-insoluble solvent is first added to the reaction liquid and, after stirring, standing and separation, the water-soluble solvent preferably containing an acid is then added to the non-aqueous layer. Alternatively, the water-soluble solvent preferably containing an acid is first added to the reaction liquid and, after stirring, standing and separation, the water-insoluble solvent is then added to the non-aqueous layer.

The acid to be added to the water-soluble solvent is not specifically limited. Examples thereof include nitric acid, hydrochloric acid, sulfuric acid, fuming sulfuric acid, boric acid, arsenic acid, phosphoric acid, prussic acid, acetic acid, peracetic acid, thioacetic acid, oxalic acid, tartaric acid, propionic acid, butyric acid, succinic acid, maleic acid, benzoic acid, anhydrous nitric acid, anhydrous sulfuric acid, boron oxide, arsenic pentoxide, phosphorus pentoxide, anhydrous chromic acid, acetic anhydride, propionic anhydride, butyric anhydride, succinic anhydride, maleic anhydride, benzoic anhydride, phthalic anhydride, silica gel, silica-alumina, and aluminum chloride, with nitric acid, hydrochloric acid, sulfuric acid, boric acid, arsenic acid, phosphoric acid, prussic acid, acetic acid, peracetic acid, thioacetic acid, oxalic acid, tartaric acid, succinic acid and maleic acid being preferred, and hydrochloric acid, sulfuric acid, boric acid, phosphoric acid and acetic acid being more preferred. These acids may be used in combination.

In place of adding the water-soluble solvent containing the acid, only the acid may be first added, followed by the addition of the water-soluble solvent. Alternatively, the water-soluble solvent is first added, followed by the addition of the acid. The water-soluble solvent containing the acid, such as an aqueous solution of acid, is added in an amount of usually 0.1 to 200 parts by weight, preferably 0.1 to 100 parts by weight, more preferably 0.1 to 50 parts by weight per 100 parts by weight of the reaction liquid. The concentration of the acid is usually 0.1 to 90%, and the pH is 6 or less, preferably 0 to 4 for achieving the effect.

It is effective for further enhancing the purity of the episulfide compound to wash the non-aqueous layer remaining after discarding the aqueous layer with water or an aqueous solution of acid. The washing is repeated usually 1 to 5 times, preferably 1 to 3 times, because an excessive number of washing increases the amount of waste liquid.

The temperature at which the water-insoluble solvent and/or the water-soluble solvent is added to the reaction liquid, and the temperature at which the aqueous solution of acid or water is added to the non-aqueous layer after removing the aqueous layer are not particularly limited as far as within the reaction temperature range, i.e., -20 to 100°C, and preferably 0 to 50°C, more preferably 0 to 30°C, most preferably 0 to 20°C. An addition temperature excessively lower than the above range would take a prolonged period of time for stabilization treatment. When the addition temperature is excessively high, the episulfide compound is degraded during the treatments to unfavorably cause coloring, etc.

Stirring is not necessarily required during the addition of the water-insoluble solvent and/or the water-soluble solvent to the reaction liquid, or during the addition of the aqueous solution of acid or water to the non-aqueous layer. The addition under stirring, however, is preferred to effect the removal of thiourea and the by-produced urea by washing with a small amount of the water-insoluble solvent, the aqueous solution of acid or water. As the stirring machine, usable are a continuous mixing/stirring machine such as a unidirectional rotary stirring machine, a reciprocating stirring machine and a static mixer, and other type stirring machines. Preferred are a unidirectional rotary stirring machine with its stirring efficiency enhanced by disposing a baffle plate in the reaction vessel and a delta wing reciprocating rotary stirring machine such as an agitor. Particularly preferred are a unidirectional rotary stirring machine with enhanced stirring efficiency such as a full zone blender and a max blender and a delta wing reciprocating rotary stirring machine with enhanced stirring efficiency such as an agitor.

It is preferred to further wash the non-aqueous layer after washed with the aqueous solution of acid, because the acid is removed to increase the purity of the episulfide compound. The remaining acid causes the haze and coloring of lens. The washing is made using a portion of water, usually 0.1 to 200 parts by weight, preferably 0.1 to 100 parts by weight, more preferably 0.1 to 50 parts by weight of water per 100 parts by weight of the reaction liquid. The washing is usually made 1 to 10 times, preferably 1 to 5 times, because excess washing results in a large amount of waste water.

In the present invention, as described above, the water-insoluble solvent and/or the water-soluble solvent is added to the reaction liquid, the resultant mixture is separated into the aqueous layer and the non-aqueous layer, and then the aqueous layer is removed. After washing as described above or without washing, the solvent is removed by distillation from the remaining non-aqueous layer to obtain the episulfide compound of the present invention.

The nitrogen content of the episulfide compound thus obtained is 5000 ppm or less, preferably 100 to 1000 ppm, more preferably 100 to 300 ppm. When the nitrogen content exceeds 5000 ppm, the resultant lens becomes colored or clouded. The nitrogen content referred to herein means the content of all nitrogen in the episulfide compound including nitrogen derived from the solvent and the starting epoxy compound in addition to nitrogen derived from thiourea and the by-produced urea.

The episulfide compound referred to in the present invention includes the compounds having, in one molecule, one or more, preferably two or more epithio structures represented by the following Formula 1: 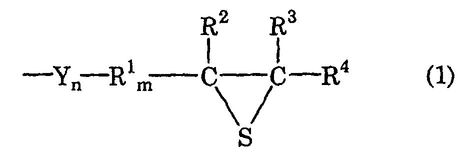 wherein R¹ is C₁-C₁₀ hydrocarbylene group; R², R³ and R⁴ are each independently C₁-C₁₀ hydrocarbyl group or hydrogen; Y is S, O, Se or Te; n is an integer of from 0 to 5; and m is 0 or 1. To attain a high refractive index, a large Abbe's number and a good balance of them, R¹ is preferably methylene or ethylene, and R², R³ and R⁴ are each preferably hydrogen or methyl. More preferably, R¹ is methylene and R², R³ and R⁴ are each hydrogen. The suffix "n" is preferably an integer from 0 to 3, more preferably 1 or 2. Y is preferably S, O or Se, more preferably S or Se.

The episulfide compounds having, in one molecule, one or more epithio structures are classified as follows:
(A) organic compounds having one or more epithio groups;
(B) organic compounds having one or more epithioalkyloxy groups;
(C) organic compounds having one or more epithioalkylthio groups;
(D) organic compounds having one or more epithioalkylseleno groups; and
(E) organic compounds having one or more epithioalkyltelluro groups.

The organic compounds A to E are mainly constituted by a chain backbone, a branched backbone, an alicyclic backbone, an aromatic backboneor a heterocyclic backbone having nitrogen, oxygen, sulfur, selenium or tellurium as the heteroatom. The organic compounds may simultaneously have, in one molecule, two or more of the epithio group, epithioalkyloxy group, epithioalkylthio group, epithioalkylseleno group and epithioalkyltelluro group. The compounds may also have a sulfide linkage, a selenide linkage, a telluride linkage, an ether linkage, a sulfone linkage, a ketone linkage, an ester linkage, an amide linkage or a urethane linkage in the molecule.
(A) Preferred examples of the organic compounds having one or more epithio groups are compounds having epoxy groups (excluding glycidyl group) with one or more epoxy groups replaced by epithio groups. Specific examples are recited below.

### (A1) Organic compounds having chain aliphatic backbone

1,1-bis(epithioethyl)methane, 1-(epithioethyl)-1-(β-epithiopropyl)methane, 1,1-bis(β-epithiopropyl)methane, 1-(epithioethyl)-1-(β-epithiopropyl)ethane, 1,2-bis(β-epithiopropyl)ethane, 1-(epithioethyl)-3-(β-epithiopropyl)butane, 1,3-bis(β-epithiopropyl)propane, 1-(epithioethyl)-4-(β-epithiopropyl)pentane, 1,4-bis(β-epithiopropyl)butane, 1-(epithioethyl)-5-(β-epithiopropyl)hexane, 1-(epithioethyl)-2-(γ-epithiobutylthio)ethane, 1-(epithioethyl)-2-[2-(γ-epithiobutylthio)ethylthio]ethane, tetrakis(β-epithiopropyl)methane, 1,1,1-tris(β-epithiopropyl)propane, 1,3-bis(β-epithiopropyl)-1-(β-epithiopropyl)-2-thiapropane and 1,5-bis(β-epithiopropyl)-2,4-bis(β-epithiopropyl)-3-thiapentane

### (A2) Compounds having alicyclic backbone

1,3- or 1,4-bis(epithioethyl)cyclohexane, 1,3- or 1,4-bis(β-epithiopropyl)cyclohexane, bis[4-(epithioethyl)cyclohexyl]methane, bis[4-(β-epithiopropyl)cyclohexyl]methane, 2,2-bis[4-(epithioethyl)-cyclohexyl]propane, 2,2-bis[4-(β-epithiopropyl)cyclohexyl]propane, bis[4-(β-epithiopropyl)cyclohexyl] sulfide, bis[4-(epithioethyl)cyclohexyl] sulfide, 2,5-bis(epithioethyl)-1,4-dithiane, 2,5-bis(β-epithiopropyl)-1,4-dithiane, 4-epithioethyl-1,2-cyclohexene sulfide, 4-epoxy-1,2-cyclohexene sulfide, 2,3-, 2,5-or 2,6-bis(1,2-epithioethyl)-1,4-diselenane, 2,3-, 2,5- or 2,6-bis(2,3-epithiopropyl)-1,4-diselenane, 2,4-, 2,5- or 2,6-bis(1,2-epithioethyl)-1,3-diselenane, 2,4-, 2,5- or 2,6-bis(2,4-epithiopropyl)-1,3-diselenane, 2,3-, 2,5-, 2,6-or 3,5-bis(1,2-epithioethyl)-1-thia-4-selenane, 2,3-, 2,5-, 2,6- or 3,5-bis(2,3-epithiopropyl)-1-thia-4-selenane, 2,4- or 4,5-bis(1,2-epithioethyl)-1,3-diselenolane, 2,4- or 4,5-bis(2,4-epithiopropyl)-1,3-diselenolane, 2,4-, 2,5- or 4,5-bis(1,2-epithioethyl)-1-thia-3-selenolane, 2,4-, 2,5- or 4,5-bis(2,4-epithiopropyl)-1-thia-3-selenolane, 2,3-, 2,4-, 2,5- or 3,4-bis(1,2-epithioethyl)selenophane, 2,3-, 2,4-, 2,5- or 3,4-bis(2,3-epithiopropyl)selenophane, 2,3-, 2,5-, 2,6-bis(1,2-epithioethyl)-1,4-ditellurane, 2,3-, 2.5- or 2,6-bis(2,3-epithiopropyl)-1,4-ditellurane, 2,4-, 2,5- or 2,6-bis(1,2-epithioethyl)-1,3-ditellurane, 2,4-, 2,5- or 2,6-bis(2,4-epithiopropyl)-1,3-ditellurane, 2,3-, 2,5-, 2,6- or 3,5-bis(1,2-epithioethyl)-1-thia-4-tellurane, 2,3-, 2,5-, 2,6- or 3,5-bis(2,3-epithiopropyl)-1-thia-4-tellurane, 2,4- or 4,5-bis(1,2-epithioethyl)-1,3-ditellurolane, 2,4- or 4,5-bis(2,4-epithiopropyl)-1,3-ditellurolane, 2,4-, 2,5- or 4,5-bis(1,2-epithioethyl)-1-thia-3-tellurolane, 2,4-, 2,5- or 4,5-bis(2,4-epithiopropyl)-1-thia-3-tellurolane, 2,3-, 2,4-, 2,5- or 3,4-bis(1,2-epithioethyl)tellurophane, and2,3-, 2,4-, 2,5- or 3,4-bis(2,3-epithiopropyl)tellurophane.

### (A3) Compounds having aromatic backbone

1,3- or 1,4-bis(epithioethyl)benzene, 1,3- or 1,4-bis(β-epithiopropyl)benzene, bis[4-(epithioethyl)phenyl]methane, bis[4-(β-epithiopropyl)phenyl]methane, 2,2-bis[4-(epithioethyl)phenyl]propane, 2,2-bis[4-(β-epithiopropyl)phenyl]propane, bis[4-(epithioethyl)phenyl] sulfide, bis[4-(β-epithiopropyl)phenyl] sulfide, bis[4-(epithioethyl)phenyl] sulfone, bis[4-(β-epithiopropyl)phenyl] sulfone, 4,4'-bis(epithioethyl)biphenyl, and 4,4'-bis(β-epithiopropyl)biphenyl.

Compounds obtained by replacing at least one hydrogen of the epithio group in the compounds A1 to A3 with methyl are also included.
(B) Preferred examples of the organic compounds having one or more epithioalkyloxy groups are epoxy compounds derived from epihalohydrin with one or more glycidyl groups replaced by epithioalkyloxy groups (thioglycidyl groups). Examples of the epoxy compounds include phenol epoxy compounds which are condensation products of epihalohydrins with polyhydric phenols such as hydroquinone, catechol, resorcinol, bisphenol A, bisphenol F, bisphenol sulfone, bisphenol ether, bisphenol sulfide, halogenated bisphenol A and novolak resins; alcohol epoxy compounds which are condensation products of epihalohydrins with polyhydric alcohols such as ethylene glycol, diethylene glycol, triethylene glycol, polyethylene glycol, propylene glycol, dipropylene glycol, polypropylene glycol, 1,3-propanediol, 1,4-butanediol, 1,6-hexanediol, neopentyl glycol, glycerol, trimethylolpropane trimethacrylate, pentaerythritol, 1,3- or 1,4-cyclohexanediol, 1,3- or 1,4-cyclohexanedimethanol, 3-selenaheptane-1,5-diol, 2,5-bis(hydroxymethyl)selenophane, 2,5-bis(4-hydroxy-2-selenabutyl)selenophane, 2,6-dihydroxymethyl-1,4-diselenane, 3,5-dihydroxymethyl-1-thia-4-selenane, 3-tellenaheptane-1,5-diol, 2,5-bis(hydroxymethyl)tellurophane, 2,5-bis(4-hydroxy-2-selenabutyl)tellurophane, 2,6-dihydroxymethyl-1,4-ditellurane, 3,5-dihydroxymethyl-1-thia-4-tellurane, hydrogenated bisphenol A, bisphenol A/ethylene oxide adducts and bisphenol A/propylene oxide adducts; glycidyl ester epoxy compounds which are condensation products of epihalohydrins with polybasic carboxylic acids such as adipic acid, sebacic acid, dodecandicarboxylic acid, dimer acid, phthalic acid, isophthalic acid, terephthalic acid, tetrahydrophthalic acid, methyltetrahydrophthalic acid, hexahydrophthalic acid, hexahydroisophthalic acid, hexahydroterephthalic acid, HET acid, nadic acid, maleic acid, succinic acid, fumaric acid, trimellitic acid, benzenetetracarboxylic acid, benzophenonetetracarboxylic acid, naphthalenedicarboxylic acid and diphenyldicarboxylic acid; amine epoxy compounds which are condensation products of epihalohydrins with primary amines such as ethylenediamine, 1,2-diaminopropane, 1,3-diaminopropane, 1,2-diaminobutane, 1,3-diaminobutane, 1,4-diaminobutane, 1,5-diaminopentane, 1,6-diaminohexane, 1,7-diaminoheptane, 1,8-diaminooctane, bis(3-aminopropyl) ether, 1,2-bis(3-aminopropoxy)ethane, 1,3-bis(3-aminopropoxy)-2,2'-dimethylpropane, 1,2-, 1,3-or 1,4-bisaminocyclohexane, 1,3- or 1,4-bisaminomethylcyclohexane, 1,3- or 1,4-bisaminoethylcyclohexane, 1,3- or 1,4-bisaminopropylcyclohexane, hydrogenated 4,4'-diaminodiphenylmethane, isophoronediamine, 1,4-bisaminopropylpiperadine, m- or p-phenylenediamine, 2,4- or 2,6-tolylenediamine, m- or p-xylylenediamine, 1,5- or 2,6-naphthalenediamine, 4,4'-diaminodiphenylmethane, 4,4'-diaminodiphenyl ether and 2,2-(4,4'-diaminodiphenyl)propane, or secondary amines such as N,N'-dimethylethylenediamine, N,N'-dimethyl-1,2-diaminopropane, N,N'-dimethyl-1,3-diaminopropane, N,N'-dimethyl-1,2-diaminobutane, N,N'-dimethyl-1,3-diaminobutane, N,N'-dimethyl-1,4-diaminobutane, N,N'-dimethyl-1,5-diaminopentane, N,N'-dimethyl-1,6-diaminohexane, N,N'-dimethyl-1,7-diaminoheptane, N,N'-diethylethylenediamine, N,N'-diethyl-1,2-diaminopropane, N,N'-diethyl-1,3-diaminopropane, N,N'-diethyl-1,2-diaminobutane, N,N'-diethyl-1,3-diaminobutane, N,N'-diethyl-1,4-diaminobutane, N,N'-diethyl-1,6-diaminohexane, piperadine, 2-methylpiperadine, 2,5- or 2,6-dimethylpiperadine, homopiperadine, 1,1-di(4-piperidyl)methane, 1,2-di(4-piperidyl)ethane, 1,3-di(4-piperidyl)propane and 1,4-di(4-piperidyl)butane; and urethane epoxy compounds produced by the reaction between the polyhydric alcohols and phenols recited above with diisocyanates and glycidol.

Examples of the compound B is recited below.

### (B1) Organic compounds having chain aliphatic backbone

bis(β-epithiopropyl) ether, bis(β-epithiopropyloxy)methane, 1,2-bis(β-epithiopropyloxy)ethane, 1,3-bis(β-epithiopropyloxy)propane, 1,2-bis(β-epithiopropyloxy)propane, 1-(β-epithiopropyloxy)-2-(β-epithiopropyloxymethyl)propane, 1,4-bis(β-epithiopropyloxy)butane, 1,3-bis(β-epithiopropyloxy)butane, 1-(β-epithiopropyloxy)-3-(β-epithiopropyloxymethyl)butane, 1,5-bis(β-epithiopropyloxy)pentane, 1-(β-epithiopropyloxy)-4-(β-epithiopropyloxymethyl)pentane, 1,6-bis(β-epithiopropyloxy)hexane, 1-(β-epithiopropyloxy)-5-(β-epithiopropyloxymethyl)hexane, 1-(β-epithiopropyloxy)-2-[(2-β-epithiopropyloxyethyl)oxy]ethane, 1-(β-epithiopropyloxy)-2-[[2-(2-β-epithiopropyloxyethyl)oxyethyl]oxy]ethane, bis(5,6-epithio-3-oxahexyl) selenide, bis(5,6-epithio-3-oxahexyl) telluride, tetrakis(β-epithiopropyloxymethyl)methane, 1,1,1-tris(β-epithiopropyloxymethyl)propane, 1,5-bis(β-epithiopropyloxy)-2-(β-epithiopropyloxymethyl)-3-thiapentane, 1,5-bis(β-epithiopropyloxy)-2,4-bis(β-epithiopropyloxymethyl)-3-thiapentane, 1-(β-epithiopropyloxy)-2,2-bis(β-epithiopropyloxymethyl)-4-thiahexane, 1,5,6-tris(β-epithiopropyloxy)-4-(β-epithiopropyloxymethyl)-3-thiahexane, 1,8-bis(β-epithiopropyloxy)-4-(β-epithiopropyloxymethyl)-3,6-dithiaoctane, 1,8-bis(β-epithiopropyloxy)-4,5-bis(β-epithiopropyloxymethyl)-3,6-dithiaoctane, 1,8-bis(β-epithiopropyloxy)-4,4-bis(β-epithiopropyloxymethyl)-3,6-dithiaoctane, 1,8-bis(β-epithiopropyloxy)-2,4,5-tris(β-epithiopropyloxymethyl)-3,6-dithiaoctane, 1,8-bis(β-epithiopropyloxy)-2,5-bis(β-epithiopropyloxymethyl)-3,6-dithiaoctane, 1,9-bis(β-epithiopropyloxy)-5-(β-epithiopropyloxymethyl)-5-[(2-β-epithiopropyloxyethyl)oxymethyl]-3,7-dithianonane, 1,10-bis(β-epithiopropyloxy)-5,6-bis[(2-β-epithiopropyloxyethyl)oxy]-3,6,9-trithiadecane, 1,11-bis(β-epithiopropyloxy)-4,8-bis(β-epithiopropyloxymethyl)-3,6,9-trithiaundecane, 1,11-bis(β-epithiopropyloxy)-5,7-bis(β-epithiopropyloxymethyl)-3,6,9-trithiaundecane, 1,11-bis(β-epithiopropyloxy)-5,7-[(2-β-epithiopropyloxyethyl)oxymethyl]-3,6,9-trithiaundecane, and 1,11-bis(β-epithiopropyloxy)-4,7-bis(β-epithiopropyloxymethyl)-3,6,9-trithiaundecane.

### (B2) Compounds having alicyclic backbone

1,3- or 1,4-bis(β-epithiopropyloxy)cyclohexane, 1,3- or 1,4-bis(β-epithiopropyloxymethyl)cyclohexane, bis[4-(β-epithiopropyloxy)cyclohexyl]methane, 2,2-bis[4-(β-epithiopropyloxy)cyclohexyl]propane, bis[4-(β-epithiopropyloxy)cyclohexyl] sulfide, 2,5-bis(β-epithiopropyloxymethyl)-1,4-dithiane, 2,5-bis(β-epithiopropyloxyethyloxymethyl)-1,4-dithiane, 2,4- or 4,5-bis(3,4-epithio-1-oxabutyl)-1,3-diselenolane, 2,4- or 4,5-bis(4,5-epithio-2-oxapentyl)-1,3-diselenolane, 2,4-, 2,5- or 4,5-bis(3,4-epithio-1-oxabutyl)-1-thia-3-selenolane, 2,4-, 2,5- or 4,5-bis(4,5-epithio-2-oxapentyl)-1-thia-3-selenolane, bis(3,4-epithio-1-oxabutyl)tricycloselenaoctane, bis(3,4-epithio-1-oxabutyl)dicycloselenanonane, 2,3-, 2,4-, 2,5- or 3,4-bis(3,4-epithio-1-oxabutyl)selenophane, 2,3-, 2,4-, 2,5- or 3,4-bis(4,5-epithio-2-oxapentyl)selenophane, 2,3-, 2,5- or 2,6-bis(3,4-epithio-1-oxabutyl)-1,4-diselenane, 2,3-, 2,5- or 2,6-bis(4,5-epithio-2-oxapenyyl)-1,4-diselenane, 2,4-, 2,5- or 2,6-bis(3,4-epithio-1-oxabutyl)-1,3-diselenane, 2,4-, 2,5- or 2,6-bis(4,5-epithio-2-oxapentyl)-1,3-diselenane, 2,3-, 2,5-, 2,6- or 3,5-bis(3,4-epithio-1-oxabutyl)-1-thia-4-selenane, 2,3-, 2,5-, 2,6- or 3,5-bis(4,5-epithio-2-oxapentyl)-1-thia-4-selenane, 2,4- or 4,5-bis(3,4-epithio-1-oxabutyl)-1,3-ditellurolane, 2,4-or 4,5-bis(4,5-epithio-2-oxapentyl)-1,3-ditellurolane, 2,4-, 2,5- or 4,5-bis(3,4-epithio-1-oxabutyl)-1-thia-3-tellurolane, 2,4-, 2,5- or 4,5-bis(4,5-epithio-2-oxapentyl)-1-thia-3-tellurolane, bis(3,4-epithio-1-oxabutyl)tricyclotelluraoctane, bis(3,4-epithio-1-oxabutyl)dicyclotelluranonane, 2,3-, 2,4-, 2,5- or 3,4-bis(3,4-epithio-1-oxabutyl)tellurophane, 2,3-, 2,4-, 2,5- or 3,4-bis(4,5-epitho-2-oxapentyl)tellurophane, 2,3-, 2,5- or 2,6-bis(3,4-epithio-1-oxabutyl)-1,4-ditellurane, 2,3-, 2,5- or 2,6-bis(4,5-epithio-2-oxapentyl)-1,4-ditellurane, 2,4-, 2,5- or 2,6-bis(3,4-epithio-1-oxabutyl)-1,3-ditellurane, 2,4-, 2,5- or 2,6-bis(4,5-epithio-2-oxapentyl)-1,3-ditellurane, 2,3-, 2,5-, 2,6- or 3,5-bis(3,4-epithio-1-oxabutyl)-1-thia-4-tellurane, and 2,3-, 2,5-, 2,6- or 3,5-bis(4,5-epithio-2-oxapentyl)-1-thia-4-tellurane.

### (B3) Compounds having aromatic backbone

1,3- or 1,4-bis(β-epithiopropyloxy)benzene, 1,3- or 1,4-bis(β-epithiopropyloxymethyl)benzene, bis[4-(β-epithiopropyl)phenyl]methane, 2,2-bis[4-(β-epithiopropylthio)phenyl]propane, bis[4-(β-epithiopropylthio)phenyl] sulfide, bis[4-(β-epithiopropylthio)phenyl] sulfone, and 4,4'-bis(β-epithiopropylthio)biphenyl.

Compounds obtained by replacing at least one hydrogen of the epithio group in the compounds B1 to B3 with methyl are also included.
(C) Preferred examples of the organic compounds having one or more epithioalkylthio groups are epoxy compounds derived from a mercapto-containing compound and an epihalohydrin with one or more epoxyalkylthio groups such as β-epoxypropylthio groups replaced by epithioalkylthio groups. Specific examples are recited below.

### (C1) Organic compounds having chain aliphatic backbone

bis(β-epithiopropyl) sulfide, bis(β-epithiopropyl) disulfide, bis(β-epithiopropyl) trisulfide, bis(β-epithiopropylthio)methane, 1,2-bis(β-epithiopropylthio)ethane, 1,3-bis(β-epithiopropylthio)propane, 1,2-bis(β-epithiopropylthio)propane, 1-(β-epithiopropylthio)-2-(β-epithiopropylthiomethyl)propane, 1,4-bis(β-epithiopropylthio)butane, 1,3-bis(β-epithiopropylthio)butane, 1-(β-epithiopropylthio)-3-(β-epithiopropylthiomethyl)butane, 1,5-bis(β-epithiopropylthio)pentane, 1-(β-epithiopropylthio)-4-(β-epithiopropylthiomethyl)pentane, 1,6-bis(β-epithiopropylthio)hexane, 1-(β-epithiopropylthio)-5-(β-epithiopropylthiomethyl)hexane, 1-(β-epithiopropylthio)-2-[(2- β-epithiopropylthioethyl)thio]ethane, 1-(β-epithiopropylthio)-2-[[2-(2-β-epithiopropylthioethyl)thioethyl]thio]ethane, tetrakis(β-epithiopropylthiomethyl)methane, 1,1,1-tris(β-epithiopropylthiomethyl)propane, 1,5-bis(β-epithiopropylthio)-2-(β-epithiopropylthiomethyl)-3-thiapentane, 1,5-bis(β-epithiopropylthio)-2,4-bis(β-epithiopropylthiomethyl)-3-thiapentane, 1-(β-epithiopropylthio)-2,2-bis(β-epithiopropylthiomethyl)-4-thiahexane, 1,5,6-tris(β-epithiopropylthio)-4-(β-epithiopropylthiomethyl)-3-thiahexane, 1,8-bis(β-epithiopropylthio)-4-(β-epithiopropylthiomethyl)-3,6-dithiaoctane, 1,8-bis(β-epithiopropylthio)-4,5-bis(β-epithiopropylthiomethyl)-3,6-dithiaoctane, 1,8-bis(β-epithiopropylthio)-4,4-bis(β-epithiopropylthiomethyl)-3,6-dithiaoctane, 1,8-bis(β-epithiopropylthio)-2,4,5-tris(β-epithiopropylthiomethyl)-3,6-dithiaoctane, 1,8-bis(β-epithiopropylthio)-2,5-bis(β-epithiopropylthiomethyl)-3,6-dithiaoctane, 1,9-bis(β-epithiopropylthio)-5-(β-epithiopropylthiomethyl)-5-[(2-β-epithiopropylthioethyl)thiomethyl]-3,7-dithianonane, 1,10-bis(β-epithiopropylthio)-5,6-bis[(2-β-epithiopropylthioethyl)thio]-3,6,9-trithiadecane, 1,11-bis(β-epithiopropylthio)-4,8-bis(β-epithiopropylthiomethyl)-3,6,9-trithiaundecane, 1,11-bis(β-epithiopropylthio)-5,7-bis(β-epithiopropylthiomethyl)-3,6,9-trithiaundecane, 1,11-bis(β-epithiopropylthio)-5,7-[(2-β-epithiopropylthioethyl)thiomethyl)-3,6,9-trithiaundecane, 1,11-bis(β-epithiopropylthio)-4,7-bis(β-epithiopropylthiomethyl)-3,6,9-trithiaundecane, tetra[2-(β-epithiopropylthio)acetylmethyl]methane, 1,1,1-tri[2-(β-epithiopropylthio)acetylmethyl]propane, tetra[2-(β-epithiopropylthiomethyl)acetylmethyl]methane, 1,1,1-tri[2-(β-epithiopropylthiomethyl)acetylmethyl]propane, bis(5,6-epithio-3-thiahexyl) selenide, 2,3-bis(6,7-thioepoxy-1-selena-4-thiaheptyl)-1-(3,4-thioepoxy-1-thiabutyl)propane, 1,1,3,3-tetrakis(4,5-thioepoxy-2-thiapentyl)-2-selenapropane, bis(4,5-thioepoxy-2-thiapentyl)-3,6,9-triselenaundecane-1,11-bis(3,4-thioepoxy-1-thiabutyl), 1,4-bis(3,4-thioepoxy-1-thiabutyl)-2,3-bis(6,7-thioepoxy-1-selena-4-thiaheptyl)butane, tris(4,5-thioepoxy-2-thiapentyl)-3-selena-6-thiaoctane-1,8-bis(3,4-thioepoxy-1-thiabutyl), bis(5,6-epithio-3-thiahexyl) telluride, 2,3-bis(6,7-thioepoxy-1-tellura-4-thiaheptyl)-1-(3,4-thioepoxy-1-thiabutyl)propane, 1,1,3,3-tetrakis(4,5-thioepoxy-2-thiapentyl)-2-tellurapropane, bis(4,5-thioepoxy-2-thiapentyl)-3,6,9-tritelleraundecane-1,11-bis(3,4-thioepoxy-1-thiabutyl), 1,4-bis(3,4-thioepoxy-1-thiabutyl)-2,3-bis(6,7-thioepoxy-1-tellura-4-thiaheptyl)butane and tris(4,5-thioepoxy-2-thiapentyl)-3-tellura-6-thiaoctane-1,8-bis(3,4-thioepoxy-1-thiabutyl).

### (C2) Compounds having alicyclic backbone

1,3- or 1,4-bis(β-epithiopropylthio)cyclohexane, 1,3- or 1,4-bis(β-epithiopropylthiomethyl)cyclohexane, bis[4-(β-epithiopropylthio)cyclohexyl]methane, 2,2-bis[4-(β-epithiopropylthio)cyclohexyl]propane, bis[4-(β-epithiopropylthio)cyclohexyl] sulfide, 2,5-bis(β-epithiopropylthiomethyl)-1,4-dithiane, 2,5-bis(β-epithiopropylthioethylthiomethyl)-1,4-dithiane, 2,3-, 2,5- or 2,6-bis(3,4-epithio-1-thiabutyl)-1,4-diselenane, 2,3-, 2,5- or 2,6-bis(4,5-epithio-2-thiapentyl)-1,4-diselenane, 2,4-, 2,5- or 5,6-bis(3,4-epithio-1-thiabutyl)-1,3-diselenane, 2,4-, 2,5-or 5,6-bis(4,5-epithio-2-thiapentyl)-1,3-diselenane, 2,3-, 2,5-, 2,6- or 3,5-bis(3,4-epithio-1-thiabutyl)-1-thia-4-selenane, 2,3-, 2,5-, 2,6- or 3,5-bis(4,5-epithio-2-thiapentyl)-1-thia-4-selenane, 2,4- or 4,5-bis(3,4-epithio-1-thiabutyl)-1,3-diselenolane, 2,4- or 4,5-bis(4,5-epithio-2-thiapentyl)-1,3-diselenolane, 2,4-, 2,5-or 4,5-bis(3,4-epithio-1-thiabutyl)-1-thia-3-selenolane, 2,4-, 2,5- or 4,5-bis(4,5-epithio-2-thiapentyl)-1-thia-3-selenolane, 2,6-bis(4,5-epithio-2-thiapentyl)-1,3,5-triselenane, bis(3,4-epithio-1-thiabutyl)-tricycloselenaoctane, bis(3,4-epithio-1-thiabutyl)dicycloselenanonane, 2,3-, 2,4-, 2,5- or 3,4-bis(3,4-epithio-1-thiabutyl)selenophane, 2,3-, 2,4-, 2,5- or 3,4-bis(4,5-epithio-2-thiapentyl)selenophane, 2-(4,5-thioepoxy-2-thiapentyl)-5-(3,4-thioepoxy-1-thiabutyl)-1-selenacyclohexane, 2,3-, 2,4-, 2,5-, 2,6-, 3,4-, 3,5-or 4,5-bis(3,4-epoxy-1-thiabutyl)-1-selenacyclohexane, 2,3-, 2,4-, 2,5-, 2,6-, 3,4-, 3,5- or 4,5-bis(4,5-thioepoxy-2-thiapentyl)-1-selenacyclohexane, 2,3-, 2,5- or 2,6-bis(3,4-epithio-1-thiabutyl)-1,4-ditellurane, 2,3-, 2,5- or 2,6-bis(4,5-epithio-2-thiapentyl)-1,4-ditellurane, 2,4-, 2,5- or 5,6-bis(3,4-epithio-1-thiabutyl)-1,3-ditellurane, 2,4-, 2,5- or 5,6-bis(4,5-epithio-2-thiapentyl)-1,3-ditellurane, 2,3-, 2,5-, 2,6- or 3,5-bis(3,4-epithio-1-thiabutyl)-1-thia-4-tellurane, 2,3-, 2,5-, 2,6- or 3,5-bis(4,5-epithio-2-thiapentyl)-1-thia-4-tellurane, 2,4- or 4,5-bis(3,4-epithio-1-thiabutyl)-1,3-ditellurolane, 2,4- or 4,5-bis(4,5-epithio-2-thiapentyl)-1,3-ditellurolane, 2,4-, 2,5- or 4,5-bis(3,4-epithio-1-thiabutyl)-1-thia-3-tellurolane, 2,4-, 2,5- or 4,5-bis(4,5-epithio-2-thiapentyl)-1-thia-3-tellurolane, 2,6-bis(4,5-epithio-2-thiapentyl)-1,3,5-tritellurane, bis(3,4-epithio-1-thiabutyl)tricyclotelluraoctane, bis(3,4-epithio-1-thiabutyl)dicyclotelluranonane, 2,3-, 2,4-, 2,5- or 3,4-bis(3,4-epithio-1-thiabutyl)tellurophane, 2,3-, 2,4-, 2,5- or 3,4-bis(4,5-epithio-2-thiapentyl)tellurophane, 2-(4,5-thioepoxy-2-thiapentyl)-5-(3,4-thioepoxy-1-thiabutyl)-1-telluracyclohexane, 2,3-, 2,4-, 2,5-, 2,6-, 3,4-, 3,5- or 4,5-bis(3,4-thioepoxy-1-thiabutyl)-1-telluracyclohexane, and 2,3-, 2,4-, 2,5-, 2,6-, 3,4-, 3,5-or 4,5-bis(4,5-thioepoxy-2-thiapentyl)-1-telluracyclohexane.

### (C3) Compounds having aromatic backbone

1,3- or 1,4-bis(β-epithiopropylthio)benzene, 1,3- or 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl] sulfide, bis[4-(β-epithiopropylthio)phenyl] sulfone, and 4,4'-bis(β-epithiopropylthio)biphenyl.

Compounds obtained by replacing at least one hydrogen of the β-epithiopropyl group of the compounds C1 to C3 with methyl are also included.
(D) Preferred examples of the organic compounds having one or more epithioalkylseleno groups are epoxy compounds with one or more epoxyalkylseleno groups such as β-epoxypropylseleno groups replaced by epithioalkylseleno groups, the epoxy compounds being derived from an epihalohdrin and a selenium compound such as alkali metal selenides, alkali metal selenols, alkyl selenols, aryl selenols and hydrogen selenide. Specific examples are recited below.

### (D1) Organic compounds having chain aliphatic backbone

bis(β-epithiopropyl) selenide, bis(β-epithiopropyl) diselenide, bis(β-epithiopropyl) triselenide, bis(β-epithiopropylseleno)methane, 1,2-bis(β-epithiopropylseleno)ethane, 1,3-bis(β-epithiopropylseleno)propane, 1,2-bis(β-epithiopropylseleno)propane, 1-(β-epithiopropylseleno)-2-(β-epithiopropylselenomethyl)propane, 1,4-bis(β-epithiopropylseleno)butane, 1,3-bis(β-epithiopropylseleno)butane, 1-(β-epithiopropylseleno)-3-(β-epithiopropylselenomethyl)butane, 1,5-bis(β-epithiopropylseleno)pentane, 1-(β-epithiopropylseleno)-4-(β-epithiopropylselenomethyl)pentane, 1,6-bis(β-epithiopropylseleno)hexane, 1-(β-epithiopropylseleno)-5-(β-epithiopropylselenomethyl)hexane, 1-(β-epithiopropylseleno)-2-[(2-β-epithiopropylselenoethyl)thio]ethane, 1-(β-epithiopropylseleno)-2-[[2-(2-β-epithiopropylselenoethyl)selenoethyl]thio]ethane, tetrakis(β-epithiopropylselenomethyl)methane, 1,1,1-tris(β-epithiopropylselenomethyl)propane, 1,5-bis(β-epithiopropylseleno)-2-(β-epithiopropylselenomethyl)-3-thiapentane, 1,5-bis(β-epithiopropylseleno)-2,4-bis(β-epithiopropylselenomethyl)-3-thiapentane, 1-(β-epithiopropylseleno)-2,2-bis(β-epithiopropylselenomethyl)-4-thiahexane, 1,5,6-tris(β-epithiopropylseleno)-4-(β-epithiopropylselenomethyl)-3-thiahexane, 1,8-bis(β-epithiopropylseleno)-4-(β-epithiopropylselenomethyl)-3,6-dithiaoctane, 1,8-bis(β-epithiopropylseleno)-4,5-bis(β-epithiopropylselenomethyl)-3,6-dithiaoctane, 1,8-bis(β-epithiopropylseleno)-4,4-bis(β-epithiopropylselenomethyl)-3,6-dithiaoctane, 1,8-bis(β-epithiopropylseleno)-2,4,5-tris(β-epithiopropylselenomethyl)-3,6-dithiaoctane, 1,8-bis(β-epithiopropylseleno)-2,5-bis(β-epithiopropylselenomethyl)-3,6-dithiaoctane, 1,9-bis(β-epithiopropylseleno)-5-(β-epithiopropylselenomethyl)-5-[(2-β-epithiopropylselenoethyl)selenomethyl]-3,7-dithianonane, 1,10-bis(β-epithiopropylseleno)-5,6-bis[(2-β-epithiopropylselenoethyl)thio]-3,6,9-trithiadecane, 1,11-bis(β-epithiopropylseleno)-4,8-bis(β-epithiopropylselenomethyl)-3,6,9-trithiaundecane, 1,11-bis(β-epithiopropylseleno)-5,7-bis(β-epithiopropylselenomethyl)-3,6,9-trithiaundecane, 1,11-bis(β-epithiopropylseleno)-5,7-[(2-β-epithiopropylselenoethyl)selenomethyl]-3,6,9-trithiaundecane, 1,11-bis(β-epithiopropylseleno)-4,7-bis(β-epithiopropylselenomethyl)-3,6,9-trithiaundecane, tetra[2-(β-epithiopropylseleno)acetylmethyl]methane, 1,1,1-tri[2-(β-epithiopropylseleno)acetylmethyl]propane, tetra[2-(β-epithiopropylselenomethyl)acetylmethyl]methane, 1,1,1-tri[2-(β-epithiopropylselenomethyl)acetylmethyl]propane, bis(5,6-epithio-3-selenohexyl) selenide, 2,3-bis(6,7-thioepoxy-1-selena-4-selenoheptyl)-1-(3,4-thioepoxy-1-selenobutyl)propane, 1,1,3,3-tetrakis(4,5-thioepoxy-2-selenopentyl)-2-selenapropane, bis(4,5-thioepoxy-2-selenopentyl)-3,6,9-triselenaundecane-1,11-bis(3,4-thioepoxy-1-selenobutyl), 1,4-bis(3,4-thioepoxy-1-selenobutyl)-2,3-bis(6,7-thioepoxy-1-selena-4-selenoheptyl)butane, tris(4,5-thioepoxy-2-selenopentyl)-3-selena-6-thiaoctane-1,8-bis(3,4-thioepoxy-1-selenobutyl), bis(5,6-epithio-3-selenohexyl) telluride, 2,3-bis(6,7-thioepoxy-1-tellura-4-selenoheptyl)-1-(3,4-thioepoxy-1-selenobutyl)propane, 1,1,3,3-tetrakis(4,5-thioepoxy-2-selenopentyl)-2-tellurapropane, bis(4,5-thioepoxy-2-selenopentyl)-3,6,9-tritelleraundecane-1,11-bis(3,4-thioepoxy-1-selenobutyl), 1,4-bis(3,4-thioepoxy-1-selenobutyl)-2.3-bis(6,7-thioepoxy-1-tellura-4-selenoheptyl)butane and tris(4,5-thiepoxy-2-selenopentyl)-3-tellura-6-thiaoctane-1,8-bis(3,4-thioepoxy-1-selenobutyl).

### (D2) Compounds having alicyclic backbone

1,3- or 1,4-bis(β-epithiopropylseleno)cyclohexane, 1,3- or 1,4-bis(β-epithiopropylselenomethyl)cyclohexane, bis[4-(β-epithiopropylseleno)cyclohexyl]methane, 2,2-bis[4-(β-epithiopropylseleno)cyclohexyl]propane, bis[4-(β-epithiopropylseleno)cyclohexyl] sulfide, 2,5-bis(β-epithiopropylselenomethyl)-1,4-dithiane, 2,5-bis(β-epithiopropylselenoethylthiomethyl)-1,4-dithiane, 2,3-, 2,5- or 2,6-bis(3,4-epithio-1-selenobutyl)-1,4-diselenane, 2,3-, 2,5- or 2,6-bis(4,5-epithio-2-selenopentyl)-1,4-diselenane, 2,4-, 2,5- or 5,6-bis(3,4-epithio-1-selenobutyl)-1,3-diselenane, 2,4-, 2,5- or 5,6-bis(4,5-epithio-2-selenopentyl)-1,3-diselenane, 2,3-, 2,5-, 2,6- or 3,5-bis(3,4-epithio-1-selenobutyl)-1-thia-4-selenane, 2,3-, 2,5-, 2,6- or 3,5-bis(4,5-epithio-2-selenopentyl)-1-thia-4-selenane, 2,4- or 4,5-bis(3,4-epithio-1-selenobutyl)-1,3-diselenolane, 2,4- or 4,5-bis(4,5-epithio-2-selenopentyl)-1,3-diselenolane, 2,4-, 2,5- or 4,5-bis(3,4-epithio-1-selenobutyl)-1-thia-3-selenolane, 2,4-, 2,5- or 4,5-bis(4,5-epithio-2-selenopentyl)-1-thia-3-selenolane, 2,6-bis(4,5-epithio-2-selenopentyl)-1,3,5-triselenane, bis(3,4-epithio-1-selenobutyl)tricycloselenaoctane, bis(3,4-epithio-1-selenobutyl)dicycloselenanonane, 2,3-, 2,4-, 2,5- or 3,4-bis(3,4-epithio-1-selenobutyl)selenophane, 2,3-, 2,4-, 2,5-, 3,4-bis(4,5-epithio-2-selenopentyl)selenophane, 2-(4,5-thioepoxy-2-selenopentyl)-5-(3,4-thioepoxy-1-selenobutyl)-1-selenacyclohexane, 2,3-, 2,4-, 2,5-, 2,6-, 3,4-, 3,5- or 4,5-bis(3,4-thioepoxy-1-selenobutyl)-1-selenacyclohexane, 2,3-, 2,4-, 2,5-, 2,6-, 3,4-, 3,5- or 4,5-bis(4,5-thioepoxy-2-selenopentyl)-1-selenacyclohexane, 2,3-, 2,5- or 2,6-bis(3,4-epithio-1-selenobutyl)-1,4-ditellurane, 2,3-, 2,5- or 2,6-bis(4,5-epithio-2-selenopentyl)-1,4-ditellurane, 2,4-, 2,5- or 5,6-bis(3,4-epithio-1-selenobutyl)-1,3-ditellurane, 2,4-, 2,5- or 5,6-bis(4,5-epithio-2-selenopentyl)-1,3-ditellurane, 2,3-, 2,5-, 2,6- or 3,5-bis(3,4-epithio-1-selenobutyl)-1-thia-4-tellurane, 2,3-, 2,5-, 2,6- or 3,5-bis(4,5-epithio-2-selenopentyl)-1-thia-4-tellurane, 2,4- or 4,5-bis(3,4-epithio-1-selenobutyl)-1,3-ditellurolane, 2,4- or 4,5-bis(4,5-epithio-2-selenopentyl)-1,3-ditellurolane, 2,4-, 2,5- or 4,5-bis(3,4-epithio-1-selenobutyl)-1-thia-3-telluroane, 2,4-, 2,5- or 4,5-bis(4,5-epithio-2-selenopentyl)-1-thia-3-tellurolane, 2,6-bis(4,5-epithio-2-selenopentyl)-1,3,5-tritellurane, bis(3,4-epithio-1-selenobutyl)tricyclotelluraoctane, bis(3,4-epithio-1-selenobutyl)dicyclotelluranonane, 2,3-, 2,4-, 2,5- or 3,4-bis(3,4-epithio-1-selenobutyl)tellurophane, 2,3-, 2,4-, 2,5- or 3,4-bis(4,5-epithio-2-selenopentyl)tellurophane, 2-(4,5-thioepoxy-2-selenopentyl)-5-(3,4-thioepoxy-1-selenobutyl)-1-telluracyclohexane, 2,3-, 2,4-, 2,5-, 2,6-, 3,4-, 3,5- or 4,5-bis(3,4-thioepoxy-1-selenobutyl)-1-telluracyclohexane, and 2,3-, 2,4-, 2,5-, 2,6-, 3,4-, 3,5- or 4,5-bis(4,5-thioepoxy-2-selenopentyl)-1-telluracyclohexane.

### (D3) Compounds having aromatic backbone

1,3- or 1,4-bis(β-epithiopropylseleno)benzene, 1,3- or 1,4-bis(β-epithiopropylselenomethyl)benzene, bis[4-(β-epithiopropylseleno)phenyl]methane, 2,2-bis[4-(β-epithiopropylseleno)phenyl]propane, bis[4-(β-epithiopropylseleno)phenyl] sulfide, bis[4-(β-epithiopropylseleno)phenyl] sulfone and 4,4'-bis(β-epithiopropylseleno)biphenyl.

Compounds obtained by replacing at least one hydrogen of the β-epithiopropyl group of the compounds D1 to D3 with methyl are also included.
(E) Preferred examples of the organic compounds having one or more epithioalkyltelluro groups are epoxy compounds with one or more epoxyalkyltelluro groups such as β-epoxypropyltelluro groups replaced by epithioalkyltelluro groups, the epoxy compounds being derived from an epihalohdrin and a tellurium compound such as alkali metal tellurides, alkali metal tellurols, alkyl tellurols, aryl tellurols and hydrogen telluride. Specific examples are recited below.

### (E1) Organic compounds having chain aliphatic backbone

bis(β-epithiopropyl) telluride, bis(β-epidithiopropyl) telluride, bis(β-epithiopropyl) ditelluride, bis(β-epidithiopropyl) ditelluride, bis(β-epithiopropyl) tritelluride, bis(β-epithiopropyltelluro)methane, 1,2-bis(β-epithiopropyltelluro)ethane, 1,3-bis(β-epithiopropyltelluro)propane, 1,2-bis(β-epithiopropyltelluro)propane, bis(epithioethyl) telluride, bis(epithioethyl) ditelluride, 1-(βepithiopropyltelluro)-2-(β-epithiopropyltelluromethyl)propane, 1,4-bis(β-epithiopropyltelluro)butane, 1,3-bis(β-epithiopropyltelluro)butane, 1-(β-epithiopropyltelluro)-3-(β-epithiopropyltelluromethyl)-butane, 1,5-bis(β-epithiopropyltelluro)pentane, 1-(β-epithiopropyltelluro)-4-(β-epithiopropyltelluromethyl)pentane, 1,6-bis(β-epithiopropyltelluro)hexane, 1-(β-epithiopropyltelluro)-5-(β-epithiopropyltelluromethyl)hexane, 1-(β-epithiopropyltelluro)-2-[(2- β-epithiopropyltelluroethyl)thio]ethane, 1-(β-epithiopropyltelluro)-2-[[2-(2-β-epithiopropyltelluroethyl)telluroethyl]thio]ethane, tetrakis(β-epithiopropyltelluromethyl)methane, 1,1,1-tris(β-epithiopropyltelluromethyl)propane, 1,5-bis(β-epithiopropyltelluro)-2-(β-epithiopropyltelluromethyl)-3-thiapentane, 1,5-bis(β-epithiopropyltelluro)-2,4-bis(β-epithiopropyltelluromethyl)-3-thiapentane, 1-(β-epithiopropyltelluro)-2,2-bis(β-epithiopropyltelluromethyl)-4-thiahexane, 1,5,6-tris(β-epithiopropyltelluro)-4-(β-epithiopropyltelluromethyl)-3-thiahexane, 1,8-bis(β-epithiopropyltelluro)-4-(β-epithiopropyltelluromethyl)-3,6-dithiaoctane, 1,8-bis(β-epithiopropyltelluro)-4,5-bis(β-epithiopropyltelluromethyl)-3,6-dithiaoctane, 1,8-bis(β-epithiopropyltelluro)-4,4-bis(β-epithiopropyltelluromethyl)-3,6-dithiaoctane, 1,8-bis(β-epithiopropyltelluro)2,4,5-tris(β-epithiopropyltelluromethyl)-3,6-dithiaoctane, 1,8-bis(β-epithiopropyltelluro)-2,5-bis(β-epithiopropyltelluromethyl)-3,6-dithiaoctane, 1,9-bis(β-epithiopropyltelluro)-5-(β-epithiopropyltelluromethyl)-5-[(2-β-epithiopropyltelluroethyl)selenomethyl]-3,7-dithianonane, 1,10-bis(β-epithiopropyltelluro)-5,6-bis[(2-β-epithiopropyltelluroethyl)thio]-3,6,9-trithiadecane, 1,11-bis(β-epithiopropyltelluro)-4,8-bis(β-epithiopropyltelluromethyl)-3,6,9-trithiaundecane, 1,11-bis(β-epithiopropyltelluro)-5,7-bis(β-epithiopropyltelluromethyl)-3,6,9-trithiaundecane, 1,11-bis(β-epithiopropyltelluro)-5,7-[(2-β-epithiopropyltelluroethyl)selenomethyl]-3,6,9-trithiaundecane, 1,11-bis(β-epithiopropyltelluro)-4,7-bis(β-epithiopropyltelluromethyl)-3,6,9-trithiaundecane, tetra[2-(β-epithiopropyltelluro)acetylmethyl]methane, 1,1,1-tri[2-(β-epithiopropyltelluro)acetylmethyl]propane, tetra[2-(β-epithiopropyltelluromethyl)acetylmethyl]methane, 1,1,1-tri[2-(β-epithiopropyltelluromethyl)acetylmethyl]propane, bis(5,6-epithio-3-tellurohexyl) selenide, 2,3-bis(6,7-thioepoxy-1-selena-4-telluroheptyl)-1-(3,4-thioepoxy-1-tellurobutyl)propane, 1,1,3,3-tetrakis(4,5-thioepoxy-2-telluropentyl)-2-selenapropane, bis(4,5-thioepoxy-2-telluropentyl)-3,6,9-triselenaundecane-1,11-bis(3,4-thioepoxy-1-tellurobutyl), 1,4-bis(3,4-thioepoxy-1-tellurobutyl)-2,3-bis(6,7-thioepoxy-1-selena-4-telluroheptyl)butane, tris(4,5-thioepoxy-2-telluropentyl)-3-selena-6-thiaoctane-1,8-bis(3,4-thioepoxy-1-tellurobutyl), bis(5,6-epithio-3-tellurohexyl) telluride, 2,3-bis(6,7-thioepoxy-1-tellura-4-telluroheptyl)-1-(3,4-thioepoxy-1-tellurobutyl)propane, 1,1,3,3-tetrakis(4,5-thioepoxy-2-telluropentyl)-2-tellurapropane, bis(4,5-thioepoxy-2-telluropentyl)-3,6,9-tritelleraundecane-1,11-bis(3,4-thioepoxy-1-tellurobutyl), 1,4-bis(3,4-thioepoxy-1-tellurobutyl)-2,3-bis(6,7-thioepoxy-1-tellura-4-telluroheptyl)butane and tris(4,5-thiepoxy-2-telluropentyl)-3-tellura-6-thiaoctane-1,8-bis(3,4-thioepoxy-1-tellurobutyl).

### (E2) Compounds having alicyclic backbone

1,3- or 1,4-bis(β-epithiopropyltelluro)cyclohexane, 1,3- or 1,4-bis(β-epithiopropyltelluromethyl)cyclohexane, bis[4-(β-epithiopropyltelluro)cyclohexyl]methane, 2,2-bis[4-(β-epithiopropyltelluro)cyclohexyl]propane, bis[4-(β-epithiopropyltelluro)cyclohexyl] sulfide, 2,5-bis(β-epithiopropyltelluromethyl)-1,4-dithiane, 2,5-bis(β-epithiopropyltelluroethylthiomethyl)-1,4-dithiane, 2,3-, 2,5- or 2,6-bis(3,4-epithio-1-tellurobutyl)-1,4-diselenane, 2,3-, 2,5- or 2,6-bis(4,5-epithio-2-telluropentyl)-1,4-diselenane, 2,4-, 2,5- or 5,6-bis(3,4-epithio-1-tellurobutyl)-1,3-diselenane, 2,4-, 2,5- or 5,6-bis(4,5-epithio-2-telluropentyl)-1,3-diselenane, 2,3-, 2,5-, 2,6- or 3,5-bis(3,4-epithio-1-tellurobutyl)-1-thia-4-selenane, 2,3-, 2,5-, 2,6- or 3,5-bis(4,5-epithio-2-telluropentyl)-1-thia-4-selenolane, 2,4- or 4,5-bis(3,4-epithio-1-tellurobutyl)-1,3-diselenolane, 2,4- or 4,5-bis(4,5-epithio-2-telluropentyl)-1,3-diselenolane, 2,4-, 2,5- or 4,5-bis(3,4-epithio-1-tellurobutyl)-1-thia-3-selenoolane, 2,4-, 2,5- or 4,5-bis(4,5-epithio-2-telluropentyl)-1-thia-3-selenolane, 2,6-bis(4,5-epithio-2-telluropentyl)-1,3,5-triselenane, bis(3,4-epithio-1-tellurobutyl)tricycloselenaoctane, bis(3,4-epithio-1-tellurobutyl)dicycloselenanonane, 2,3-, 2,4-, 2,5- or 3,4-bis(3,4-epithio-1-tellurobutyl)selenophane, 2,3-, 2,4-, 2,5- or 3,4-bis(4,5-epithio-2-telluropentyl)selenophane, 2-(4,5-thioepoxy-2-telluropentyl)-5-(3,4-thioepoxy-1-tellurobutyl)-1-selenacyclohexane, 2,3-, 2,4-, 2,5-, 2,6-, 3,4-, 3,5- or 4,5-bis(3,4-thioepoxy-1-tellurobutyl)-1-selenacyclohexane, 2,3-, 2,4-, 2,5-, 2,6-, 3,4-, 3,5- or 4,5-bis(4,5-thioepoxy-2-telluropentyl)-1-selenacyclohexane, 2,3-, 2,5- or 2,6-bis(3,4-epithio-1-tellurobutyl)-1,4-ditellurane, 2,3-, 2,5- or 2,6-bis(4,5-epithio-2-telluropentyl)-1,4-ditellurane, 2,4-, 2,5- or 5,6-bis(3,4-epithio-1-tellurobutyl)-1,3-ditellurane, 2,4-, 2,5- or 5,6-bis(4,5-epithio-2-telluropentyl)-1,3-ditellurane, 2,3-, 2,5-, 2,6- or 3,5-bis(3,4-epithio-1-tellurobutyl)-1-thia-4-tellurane, 2,3-, 2,5-, 2,6- or 3,5-bis(4,5-epithio-2-telluropentyl)-1-thia-4-tellurane, 2,4- or 4,5-bis(3,4-epithio-1-tellurobutyl)-1,3-ditellurolane, 2,4- or 4,5-bis(4,5-epithio-2-telluropentyl)-1,3-ditellurolane, 2,4-, 2,5- or 4,5-bis(3,4-epithio-1-tellurobutyl)-1-thia-3-tellurolane, 2,4-, 2,5- or 4,5-bis(4,5-epithio-2-telluropentyl)-1-thia-3-tellurolane, 2,6-bis(4,5-epithio-2-telluropentyl)-1,3,5-tritellurane, bis(3,4-epithio-1-tellurobutyl)tricyclotelluraoctane, bis(3,4-epithio-1-tellurobutyl)dicyclotelluranonane, 2,3-, 2,4-, 2,5- or 3,4-bis(3,4-epithio-1-tellurobutyl)tellurophane, 2,3-, 2,4-, 2,5- or 3,4-bis(4,5-epithio-2-telluropentyl)tellurophane, 2-(4,5-thioepoxy-2-telluropentyl)-5-(3,4-thioepoxy-1-tellurobutyl)-1-telluracyclohexane, 2,3-, 2,4-, 2,5-, 2,6-, 3,4-, 3,5- or 4,5-bis(3,4-thioepoxy-1-tellurobutyl)-1-telluracyclohexane, and 2,3-, 2,4-, 2,5-, 2,6-, 3,4-, 3,5- or 4,5-bis(4,5-thioepoxy-2-telluropentyl)-1-telluracyclohexane

### (E3) Compounds having aromatic backbone

1,3- or 1,4-bis(β-epithiopropyltelluro)benzene, 1,3- or 1,4-bis(β-epithiopropyltelluromethyl)benzene, bis[4-(β-epithiopropyltelluro)phenyl]methane, 2,2-bis[4-(β-epithiopropyltelluro)phenyl]propane, bis[4-(β-epithiopropyltelluro)phenyl] sulfide, bis[4-(β-epithiopropyltelluro)phenyl] sulfone, and 4,4'-bis(β-epithiopropyltelluro)biphenyl.

Compounds obtained by replacing at least one hydrogen of the β-epithiopropyl group of the compounds E1 to E3 with methyl are also included.

Further the compounds A to E include organic compounds having unsaturated groups. Examples thereof include vinylphenyl thioglycidyl ether, vinylbenzyl thioglycidyl ether, thioglycidyl methacrylate, thioglycidyl acrylate and allyl thioglycidyl ether.

Examples of the episulfide compounds having another epithio group include ethylene sulfide, propylene sulfide, thioglycidol, thioglycidyl esters of monocarboxylic acid such as acetic acid, propionic acid, and benzoic acid, and thioglycidyl ether such as methyl thioglycidyl ether, ethyl thioglycidyl ether, propyl thioglycidyl ether and butyl thioglycidyl ether.

Of the above episulfide compounds, preferred are the organic compound B having one or more epithioalkyloxy groups, the organic compound C having one or more epithioalkylthio groups and the organic compound D having one or more epithioalkylseleno groups, with the organic compounds C and D being more preferred. Specific examples of more preferred episulfide compounds include bis(β-epithiopropyl) sulfide, bis(β-epithiopropyl) disulfide, bis(β-epithiopropyl) selenide, bis(β-epithiopropyl) diselenide, and chain, branched, alicyclic, aromatic or heterocyclic compounds having two or more β-epithiopropylthio groups or β-epithiopropylseleno groups, which are listed above. Particularly preferred are chain compounds having two or more β-epithiopropylthio groups or β-epithiopropylseleno groups, bis(β-epithiopropyl) sulfide, bis(β-epithiopropyl) disulfide, bis(β-epithiopropyl) selenide, and bis(β-epithiopropyl) diselenide.

The epoxy compound used in the present invention includes all epoxy compounds having, in one molecule, one or more structures represented by Formula 2, more specifically, includes epoxy compounds corresponding to the above episulfide compounds, i.e., those obtained by replacing the episulfide group of the above episulfide compounds with the epoxy group.

The episulfide compound of the present invention is cured by polymerization to the optical material, solely or after mixed with a compound having one or more functional groups that are reactive to the epithio group of the episulfide compound, a compound having one or more such functional groups and one or more monopolymerizable functional groups, a compound having one or more such monopolymerizable functional groups, or a compound having one functional group that is reactive to the epithio group and monopolymerizable. Examples of such compound to be mixed are proposed in Japanese Patent Application Laid-Open Nos. 9-71580, 9-110979 and 9-255781.

As the curing catalyst for use in curing the episulfide compound by polymerization, usable are amines, quaternary ammonium salts, quaternary phosphonium salts, phosphines, mineral acid, Lewis acids, organic acids, silicic acids and boron tetrafluoride which are described in Japanese Patent Application Laid-Open Nos. 10-28481 and 10-370222.

In curing the unsaturated episulfide compound, it is preferred to use a radical polymerization initiator as a polymerization promoter. The radical polymerization initiator is not particularly limited as far as it generates radicals by heating or irradiation with ultraviolet light or electron beam, and examples thereof are described in Japanese Patent Application Laid-Open Nos. 9-71580, 9-110979 and 9-255781.

In the production of the optical material, known additives such as anti-oxidant and ultraviolet absorber may be added to enhance the practicality of the optical material. The optical material of the present invention is somewhat easily separated from a mold during the polymerization. It is preferred, if necessary, to enhance the adhesion of the curing product to the mold by a known external or internal adhesion improver.

To make the optical material sufficiently resistant to oxidization, an anti-oxidizing compound having one or more SH groups may be added solely or in combination with known anti-oxidant. The compound having one or more SH groups may include mercaptans and thiophenols, each optionally having an unsaturated group such as vinyl, aromatic vinyl, methacryl, acryl and allyl. Examples thereof are described in Japanese Patent Application Laid-Open Nos. 9-71580, 9-110979, 9-255781 and 10-298287.

To improve the dyeability and mechanical-strength, a compound having one or more active hydrogen atoms other than SH hydrogen may be added. Examples thereof are described in Japanese Patent Application Laid-Open Nos. 9-71580, 9-110979, 9-255781 and 11-166037.

In the present invention, the episulfide compound or a composition containing the episulfide compound is injected into a mold made of glass or metal after uniformly blended with a catalyst, an adhesion improver, an anti-oxidant, an ultraviolet absorber, a radical polymerization initiator or another additive for improving various properties. Then, cured by polymerization under heating and released from the mold to obtain the optical material.

The episulfide compound or the composition containing the episulfide compound may be injected into the mold after prepolymerizing a part or whole part thereof at -100 to 160°C for 0.1 to 72 h prior to the injection in the presence or absence of a catalyst with or without stirring. The prepolymerization is carried out preferably at -10 to 100°C for 1 to 48 h, more preferably at 0 to 60°C for 1 to 48 h.

The production of the optical material by polymerization curing the episulfide compound of the present invention will be described in more detail. As described above, the episulfide compound is injected into a mold solely or after blended with a supplementary material, and cured to the optical material. A mixture of the episulfide compound or a composition containing the episulfide compound with a catalyst may be added with, if necessary, a compound having two or more functional groups that are reactive to the epithio group, a compound having one or more such functional groups and one or more monopolymerizable functional groups, a compound having one or more such monopolymerizable functional groups, or a compound having one functional group that is reactive to the epithio group and monopolymerizable, and/or additives selected from anti-oxidants, improvers for dyeability and mechanical strength, adhesion improvers, stabilizers, radical polymerization initiators, etc. The episulfide compound, the catalyst, the optional compounds and the additives may be all blended simultaneously in the same container, or may be blended by adding each component stepwise. Alternatively, a few of the components are separately blended and then blended together in the same container. The order of the blending is not particularly limited.

The mixing temperature and time are not critical as far as the components are sufficiently mixed. An excessively high temperature and an excessively long mixing time unfavorably make the injection operation difficult because undesirable reaction between the starting materials and additives is induced to increase the viscosity. The mixing temperature is about -50 to 100°C, preferably -30 to 50°C, more preferably -5 to 30°C. The mixing time is one minute to five hours, preferably five minutes to two hours, more preferably 5 to 30 min, and most preferably 5 to 15 min. The degasification under reduced pressure prior to the mixing, during the mixing or after the mixing of the starting materials and additives is preferred to prevent the generation of bubbles during the subsequent injection step and curing step by polymerization. The degree of evacuation is about 10 Pa to 100 kPa, preferably 1000 Pa to 40 kPa. To increase the quality of the optical material of the invention, it is preferred to remove impurities by filtering the starting materials before or after mixing through a filter having a pore size of about 0.05 to 3 µm.

After injecting the starting mixture into a glass or meal mold, the curing by polymerization is conducted using an electric furnace. The curing time is 0.1 to 100 h, preferably 1 to 48 h. The curing temperature is -10 to 160°C, preferably -10 to 140°C. The polymerization is carried out by keeping the starting mixture at a given polymerization temperature for a given period of time while raising the temperature at 0.1 to 100°C/h, lowering the temperature at 0.1 to 100°C/h or using a combination thereof. After curing, it is preferred to anneal the optical material at 50 to 150°C for 10 min to 5 h because the strain of the optical material can be removed. The optical material may be further subjected to surface treatment for improving dyeability, providing hard coating, and imparting non-reflection and non-fogging properties, etc.

The optical material of the invention thus obtained has a refractive index of 1.65 to 1.75, Abbe's number of 30 to 40, and a yellowness, a measure of coloring, of 0.3 to 1.5 in terms of YI value.

The present invention will be described in more detail by reference to the following examples which should not be construed to limit the scope of the invention thereto. The nitrogen content of the obtained episulfide compound was measured by a total nitrogen microanalyzer. The properties of the polymerization-cured product were measured by the following methods.

### Nitrogen Content:

Each sample (1 g) was diluted with 50 ml of toluene, followed by quantitative analysis using a total nitrogen microanalyzer.

### Refractive Index and Abbe's Number:

Measured at 25°C using Abbe refractometer.

### Haze:

The degree of haze of lens was visually observed in a darkroom under fluorescent light. A lens with no haze is preferred.

### Yellowness:

YI value of lens was measured by a spectrocolorimeter. YI value of 1.5 or less is preferred, and YI value of 1.0 or less is more preferred.

### EXAMPLE 1

Into a flask equipped with a delta wing reciprocating rotary stirring machine (Agitor, trademark of Shimadzu Corporation), a thermometer and an nitrogen inlet, were charged 425.5 g (2.5 mol) of 1,2-bis(β-epoxypropylthio)ethane, 761.2 g (10 mol) of thiourea, 43.9 g (0.43 mol) of acetic anhydride, 2.3 kg of toluene, and 2.1 kg of methanol. The mixture was allowed to react at 30°C for 8 h. After the reaction, 2.3 kg of toluene and 0.3 kg of a 25% aqueous solution of sulfuric acid were added. The mixture was cooled to 15°C and stirred for 20 min. After the stirring was stopped, the separated aqueous layer was discarded. After adding 0.3 kg of water to the non-aqueous layer, the mixture was stirred at 15°C for 20 min. After the stirring was stopped, the aqueous layer was discarded. This water addition-stirring-separation operation was repeated three times. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 2

The procedure of Example 1 was repeated except for using 2.5 mol of 2,6-dimethyl-1,2:6,7-diepoxy-4-thiaheptane in place of 1,2-bis(β-epoxypropylthio)ethane to obtain 2,6-dimethyl-1,2:6,7-diepithio-4-thiaheptane. The nitrogen content thereof is shown in Table 1.

Then, 100 parts by weight of 2,6-dimethyl-1,2:6,7-diepithio-4-thiaheptane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.70 and Abbe's number of 36. The results of observing the appearance showed that the lens was free from haze and colorless. The results are shown in Table 1.

### EXAMPLE 3

The procedure of Example 1 was repeated except for using 2.5 mol of bis(β-epoxypropyl) sulfide in place of 1,2-bis(β-epoxypropylthio)ethane to obtain bis(β-epithiopropyl) sulfide. The nitrogen content thereof is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 4

The procedure of Example 1 was repeated except for using 2.5 mol of phenyl glycidyl ether in place of 1,2-bis(β-epoxypropylthio)ethane to obtain phenyl thioglycidyl ether. The nitrogen content thereof is shown in Table 1.

Then, 100 parts by weight of phenyl thioglycidyl ether thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.63 and Abbe's number of 38. The results of observing the appearance showed that the lens was free from haze and colorless. The results are shown in Table 1.

### EXAMPLE 5

The procedure of Example 1 was repeated except for using 2.5 mol of 1,2,3-tris(β-epoxypropyl)propane in place of 1,2-bis(β-epoxypropylthio)ethane to obtain 1,2,3-tris(β-epithiopropyl)propane. The nitrogen content thereof is shown in Table 1.

Then, 100 parts by weight of 1,2,3-tris(β-epithiopropyl)propane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.70 and Abbe's number of 37. The results of observing the appearance showed that the lens was free from haze and colorless. The results are shown in Table 1.

### EXAMPLE 6

Into a flask equipped with a full zone stirring machine, a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 0.9 kg of toluene, and 3.3 kg of methanol. The mixture was allowed to react at 30°C for 10 h. After the reaction, 6.3 kg of toluene was added. The mixture was cooled to 15°C and stirred for 20 min. After the stirring was stopped, the separated aqueous layer was discarded. After adding 0.3 kg of a 10% aqueous solution of sulfuric acid, the stirring was continued at 15°C for 20 min. After the stirring was stopped, the aqueous layer was discarded. After adding 0.3 kg of water to the non-aqueous layer, the mixture was stirred at 15°C for 20 min. After the stirring was stopped, the aqueous layer was discarded. This water addition-stirring-separation operation was repeated four times. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 7

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 1.6 kg of toluene, and 2.7 kg of methanol. The mixture was allowed to react at 20°C for 9 h. After the reaction, 4.3 kg of toluene and 0.5 kg of a 10% aqueous solution of sulfuric acid were added. The mixture was cooled to 15°C and stirred for 20 min. After the stirring was stopped, the separated aqueous layer was discarded. After adding 0.5 kg of a 0.5% aqueous solution of sulfuric acid, the stirring was continued at 15°C for 20 min. After the stirring was stopped, the separated aqueous layer was discarded. After adding 0.5 kg of water to the non-aqueous layer, the mixture was stirred at 15°C for 20 min. After the stirring was stopped, the aqueous layer was discarded. This water addition-stirring-separation operation was repeated three times. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight oftetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 8

The procedure of Example 7 was repeated except for using a 10% aqueous solution of phosphorus acid in stead of the 10% aqueous solution of sulfuric acid, and using a 0.5% aqueous solution of phosphorus acid in stead of the 0.5% aqueous solution of sulfuric acid, thereby obtaining bis(β-epithiopropyl) sulfide. The nitrogen content thereof is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 9

The procedure of Example 7 was repeated except for using a 10% aqueous solution of acetic acid in stead of the 10% aqueous solution of sulfuric acid, and using a 0.5% aqueous solution of acetic acid in stead of the 0.5% aqueous solution of sulfuric acid, thereby obtaining bis(β-epithiopropyl) sulfide. The nitrogen content thereof is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 10

Into a flask equipped with a max blend stirring machine, a thermometer and an nitrogen inlet, were charged 425.5 g (2.5 mol) of 1,2-bis(β-epoxypropylthio)ethane, 761.2 g (10 mol) of thiourea, 43.9 g (0.43 mol) of acetic anhydride, 2.9 kg of toluene, and 1.5 kg of methanol. The mixture was allowed to react at 24°C for 16 h. After the reaction, 0.1 kg of toluene and 1.0 kg of water were added. The mixture was cooled to 10°C and stirred for 20 min. After the stirring was stopped, the separated aqueous layer was discarded. After adding 1.0 kg of a 30% aqueous solution of sulfuric acid, the stirring was continued at 15°C for 20 min. After the stirring was stopped, the separated aqueous layer was discarded. After adding 1.0 kg of water, the mixture was stirred at 15°C for 20 min. After the stirring was stopped, the aqueous layer was discarded. This water addition-stirring-separation operation was repeated three times. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 11

Into a flask equipped with a full zone stirring machine, a thermometer and an nitrogen inlet, were charged 425.5 g (2.5 mol) of 1,2-bis(β-epoxypropylthio)ethane, 761.2 g (10 mol) of thiourea, 43.9 g (0.43 mol) of acetic anhydride, 3.6 kg of dichloromethane, and 1.6 kg of ethanol. The mixture was allowed to react at 24°C for 16 h. After the reaction, 5.6 kg of dichloromethane and 10.0 kg of a 30% aqueous solution of sulfuric acid were added. The mixture was cooled to 10°C and stirred for 20 min. After the stirring was stopped, the separated aqueous layer was discarded. Then, by removing dichloromethane by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 12

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 41.0 g (0.4 mol) of acetic anhydride, 1.6 kg of toluene, and 2.7 kg of methanol. The mixture was allowed to react at 20°C for 9 h. After the reaction, 4.3 kg of water was added. The mixture was cooled to 10°C and stirred for 20 min. After the stirring was stopped, the separated aqueous layer was discarded. After adding 10.0 kg of water, the stirring was continued at 15°C for 20 min. After the stirring was stopped, the separated aqueous layer was discarded. This water addition-stirring-separation operation was repeated seven times. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 13

Into a flask equipped with a turbine impeller stirring machine (with baffle plate), a thermometer and an nitrogen inlet, were charged·425.5 g (2.5 mol) of 1,2-bis(β-epoxypropylthio)ethane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, 2.3 kg of water was added. The mixture was cooled to 10°C and stirred for 20 min. After the stirring was stopped, the separated aqueous layer was discarded. After adding 0.5 kg of a 10% hydrochloric acid, the mixture was stirred at 15°C for 20 min. After the stirring was stopped, the separated aqueous layer was discarded. This hydrochloric acid addition-stirring-separation operation was repeated five times. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 14

Into a flask equipped with a delta wing reciprocating rotary stirring machine (Agitor, trademark of Shimadzu Corporation), a thermometer and an nitrogen inlet, were charged 425.5 g (2.5 mol) of 1,2-bis(β-epoxypropylthio)ethane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, the mixture was cooled to 10°C and added with 11.0 kg of toluene, followed by stirring at 10°C for 20 min. After the stirring was stopped, the separated aqueous layer was discarded. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 15

Into a flask equipped with a propeller stirring machine (with no baffle plate), a thermometer and an nitrogen inlet, were charged 530.0 g (2.5 mol) of 1,2,3-tris(β-epoxypropyl)propane, 761.2 g (10 mol) of thiourea, 41.0 g (0.4 mol) of acetic anhydride, 2.3 kg of toluene, and 2.1 kg of methanol. The mixture was allowed to react at 20°C for 9 h. After the reaction, 5.3 kg of toluene and 3.1 kg of a 10% aqueous solution of sulfuric acid were added. The mixture was cooled to 15°C and stirred for 20 min. After the stirring was stopped, the separated aqueous layer was discarded. After adding 3.1 kg of water, the mixture was stirred at 15°C for 20 min. After the stirring was stopped, the separated aqueous layer was discarded. Then, by removing toluene by distillation, 1,2,3-tris(β-epithiopropyl)propane was obtained. The nitrogen content is shown in Table 1.

Then, 100 parts by weight of 1,2,3-tris(β-epithiopropyl)propane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.70 and Abbe's number of 37. The results of observing the appearance showed that the lens was free from haze and colorless. The results are shown in Table 1.

### EXAMPLE 16

Into a flask equipped with a delta wing reciprocating rotary stirring machine (Agitor, trademark of Shimadzu Corporation), a thermometer and an nitrogen inlet, were charged 425.5 g (2.5 mol) of 1,2-bis(β-epoxypropylthio)ethane, 761.2 g (10 mol) of thiourea, 43.9 g (0.43 mol) of acetic anhydride, 2.3 kg of toluene, and 2.1 kg of methanol. The mixture was allowed to react at 30°C for 8 h. After the reaction, 0.3 kg of a 25% aqueous solution of sulfuric acid was added. The mixture was cooled to 15°C and stirred for 20 min. After the stirring was stopped, the separated aqueous layer was discarded. After adding 0.3 kg of water, the mixture was stirred at 15°C for 20 min. After the stirring was stopped, the separated aqueous layer was discarded. This water addition-stirring-separation operation was repeated three times. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 17

The procedure of Example 16 was repeated except for using 2.5 mol of 2,6-dimethyl-1,2:6,7-diepoxy-4-thiaheptane in place of 1,2-bis(β-epoxypropylthio)ethane to obtain 2,6-dimethyl-1,2:6,7-diepithio-4-thiaheptane. The nitrogen content thereof is shown in Table 1.

Then, 100 parts by weight of 2,6-dimethyl-1,2:6,7-diepithio-4-thiaheptane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.70 and Abbe's number of 36. The results of observing the appearance showed that the lens was free from haze and colorless. The results are shown in Table 1.

### EXAMPLE 18

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 1.6 kg of toluene, and 2.7 kg of methanol. The mixture was allowed to react at 20°C for 9 h. After the reaction, 0.5 kg of a 10% aqueous solution of sulfuric acid was added. The mixture was cooled to 15°C and stirred for 20 min. After the stirring was stopped, the separated aqueous layer was discarded. After adding 0.5 kg of a 0.5% aqueous solution of sulfuric acid, the stirring was continued at 15°C for 20 min. After the stirring was stopped, the separated aqueous layer was discarded. After adding 0.5 kg of water, the mixture was stirred at 15°C for 20 min. After the stirring was stopped, the aqueous layer was discarded. This water addition-stirring-separation operation was repeated three times. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 19

The procedure of Example 18 was repeated except for using a 10% aqueous solution of phosphorus acid in stead of the 10% aqueous solution of sulfuric acid, and using a 0.5% aqueous solution of phosphorus acid in stead of the 0.5% aqueous solution of sulfuric acid, thereby obtaining bis(β-epithiopropyl) sulfide. The nitrogen content thereof is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 20

Into a flask equipped with a delta wing reciprocating rotary stirring machine (Agitor, trademark of Shimadzu Corporation), a thermometer and an nitrogen inlet, were charged 425.5 g (2.5 mol) of 1,2-bis(β-epoxypropylthio)ethane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, the mixture was cooled to 10°C and added with 5.4 kg of toluene and 3.1 kg of water, followed by stirring at 10°C for 20 min. After the stirring was stopped, the separated aqueous layer was discarded. After adding 5.0 kg of a 1% aqueous solution of sulfuric acid, the mixture was stirred at 15°C for 20 min. After the stirring was stopped, the aqueous layer was discarded. This addition of 1% sulfuric acid solution-stirring-separation operation was repeated four times. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 21

Into a flask equipped with a delta wing reciprocating rotary stirring machine (Agitor, trademark of Shimadzu Corporation), a thermometer and an nitrogen inlet, were charged 425.5 g (2.5 mol) of 1,2-bis(β-epoxypropylthio)ethane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, the mixture was cooled to 10°C and added with 10.0 kg of a 50% aqueous solution of sulfuric acid, followed by stirring at 10°C for 20 min. After the stirring was stopped, the separated aqueous layer was discarded. Then, by removing toluene by distillation, 1,2-bis(β-epithiopropylthio)ethane was obtained. The nitrogen content is shown in Table 1.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 22

Into a flask equipped with a full zone stirring machine, a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 0.9 kg of toluene, and 3.3 kg of methanol. The mixture was allowed to react at 30°C for 10 h. After the reaction, 6.3 kg of toluene was added. The mixture was cooled to 15°C and stirred for 20 min. After the stirring was stopped, the separated aqueous layer was discarded. After adding 0.3 kg of water, the stirring was continued at 15°C for 20 min. After the stirring was stopped, the aqueous layer was discarded. This water addition-stirring-separation operation was repeated ten times. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 23

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 1.6 kg of toluene, and 2.7 kg of methanol. The mixture was allowed to react at 20°C for 9 h. After the reaction, 10.0 kg of toluene and 10.0 kg of water were added. The mixture was cooled to 15°C and stirred for 60 min. After the stirring was stopped, the separated aqueous layer was discarded. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 24

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 43.9 g (0.43 mol) of acetic anhydride, 1.6 kg of toluene, and 2.7 kg of methanol. The mixture was allowed to react at 20°C for 9 h. After the reaction, 10.0 kg of water was added. The mixture was cooled to 15°C and stirred for 20 min. After the stirring was stopped, the separated aqueous layer was discarded. Then, by removing toluene by distillation, bis(β-epithiopropyl) sulfide was obtained. The nitrogen content is shown in Table 1.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze and colorless. The results are shown in Table 1.

### EXAMPLE 25

Into a flask equipped with a delta wing reciprocating rotary stirring machine (Agitor, trademark of Shimadzu Corporation), a thermometer and an nitrogen inlet, were charged 445.0 g (2.5 mol) of bis(β-epoxypropyl) disulfide, 761.2 g (10 mol) of thiourea, 43.9 g (0.43 mol) of acetic anhydride, 1.6 kg of toluene, and 2.7 kg of methanol. The mixture was allowed to react at 20°C for 9 h. After the reaction, 4.3 kg of toluene and 0.5 kg of a 10% aqueous solution of sulfuric acid were added. The mixture was cooled to 15°C and stirred for 20 min. After the stirring was stopped, the separated aqueous layer was discarded. After adding 0.5 kg of a 5% aqueous solution of sulfuric acid, the mixture was stirred at 15°C for 20 min. After the stirring was stopped, the aqueous layer was discarded. After further adding 0.5 kg of water, the mixture was stirred at 15°C for 20 min. After the stirring was stopped, the aqueous layer was discarded. This water addition-stirring-separation operation was repeated three times. Then, by removing toluene by distillation, bis(β-epithiopropyl) disulfide was obtained. The nitrogen content is shown in Table 1.

Then, 100 parts by weight of bis(β-epithiopropyl) disulfide thus obtained was blended with 1.0 part by weight of triphenylphosphine, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.74 and Abbe's number of 34. The results of observing the appearance showed that the lens was free from haze and colorless. The results are shown in Table 1.

### EXAMPLE 26

The procedure of Example 1 was repeated except for using 2.5 mol of bis(β-epoxypropyl)selenide in place of 1,2-bis(β-epoxypropylthio)ethane to obtain bis(β-epithiopropyl)selenide. The nitrogen content thereof is shown in Table 1.

Then, 100 parts by weight of bis(β-epithiopropyl)selenide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.73 and Abbe's number of 33. The results of observing the appearance showed that the lens was free from haze and colorless. The results are shown in Table 1.

**Table 1**

| Examples | Episulfide Compound | | Lens | |
|---|---|---|---|---|
| | Name | Nitrogen Content (ppm) | Haze | Yellowing (YI value) |
| 1 | 1,2-bis(β-epithiopropylthio)ethane | 180 | none | 0.49 |
| 2 | 2,6-dimethyl-1,2:6,7-diepithio-4-thiaheptane | 210 | none | 0.61 |
| 3 | bis(β-epithiopropyl)sulfide | 248 | none | 0.59 |
| 4 | phenyl thioglycidyl ether | 168 | none | 0.58 |
| 5 | 1,2,3-tris(β-epithiopropylthio)propane | 195 | none | 0.60 |
| 6 | bis(β-epithiopropyl)sulfide | 323 | none | 0.65 |
| 7 | bis(β-epithiopropyl)sulfide | 164 | none | 0.49 |
| 8 | bis(β-epithiopropyl)sulfide | 213 | none | 0.59 |
| 9 | bis(β-epithiopropyl)sulfide | 377 | none | 0.88 |
| 10 | 1,2-bis(β-epithiopropylthio)ethane | 182 | none | 0.48 |
| 11 | 1,2-bis(β-epithiopropylthio)ethane | 523 | none | 0.90 |
| 12 | bis(β-epithiopropyl)sulfide | 3480 | none | 1.31 |
| 13 | 1,2-bis(β-epithiopropylthio)ethane | 967 | none | 1.06 |
| 14 | 1,2-bis(β-epithiopropylthio)ethane | 4971 | none | 1.49 |
| 15 | 1,2,3-tris(β-epithiopropylthio)propane | 267 | none | 0.58 |
| 16 | 1,2-bis(β-epithiopropylthio)ethane | 212 | none | 0.53 |
| 17 | 2,6-dimethyl-1,2:6,7-diepithio-4-thiaheptane | 232 | none | 0.59 |
| 18 | bis(β-epithiopropyl)sulfide | 150 | none | 0.49 |
| 19 | bis(β-epithiopropyl)sulfide | 230 | none | 0.54 |
| 20 | 1,2-bis(β-epithiopropylthio)ethane | 2246 | none | 1.21 |
| 21 | 1,2-bis(β-epithiopropylthio)ethane | 203 | none | 0.60 |
| 22 | bis(β-epithiopropyl)sulfide | 3195 | none | 1.36 |
| 23 | bis(β-epithiopropyl)sulfide | 4178 | none | 1.43 |
| 24 | bis(β-epithiopropyl)sulfide | 4159 | none | 1.43 |
| 25 | bis(β-epithiopropyl)disulfide | 209 | none | 1.04 |
| 26 | bis(β-epithiopropyl)selenide | 341 | none | 1.49 |

### COMPARATIVE EXAMPLE 1

Into a flask equipped with a delta wing reciprocating rotary stirring machine (Agitor, trademark of Shimadzu Corporation), a thermometer and an nitrogen inlet, were charged 365.5 g (2.5 mol) of bis(β-epoxypropyl) sulfide, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, 0.3 kg of water was added. The mixture was cooled to 15°C and stirred for 20 min. After the stirring was stopped, the separated aqueous layer was discarded. Then, by removing toluene by distillation, bis(β-epithiopropyl) sulfide was obtained. The nitrogen content is shown in Table 2.

Then, 100 parts by weight of bis(β-epithiopropyl) sulfide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 36. The results of observing the appearance showed that the lens was free from haze, but colored in yellow. The results are shown in Table 2.

### COMPARATIVE EXAMPLE 2

Into a flask equipped with a full zone stirring machine, a thermometer and an nitrogen inlet, were charged 425.5 g (2.5 mol) of 1,2-bis(β-epoxypropylthio)ethane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, 0.3 kg of water was added. The mixture was cooled to 15°C and stirred for 20 min. After the stirring was stopped, the separated aqueous layer was discarded. After further adding 0.3 kg of water, the stirring was continued at 15°C for 20 min. After the stirring was stopped, the separated aqueous layer was discarded. Then, by removing toluene by distillation, 1,2-bis(β-epithiopropylthio)ethane was obtained. The nitrogen content is shown in Table 2.

Then, 100 parts by weight of 1,2-bis(β-epithiopropylthio)ethane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.71 and Abbe's number of 35. The results of observing the appearance showed that the lens was free from haze, but colored in yellow. The results are shown in Table 2.

### COMPARATIVE EXAMPLE 3

Into a flask equipped with a max blend stirring machine, a thermometer and an nitrogen inlet, were charged 375.0 g (2.5 mol) of phenyl glycidyl ether, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, 2.3 kg of toluene and 0.3 kg of water. The mixture was cooled to 15°C and stirred for 20 min. After the stirring was stopped, the separated aqueous layer was discarded. Then, by removing toluene by distillation, phenyl thioglycidyl ether was obtained. The nitrogen content is shown in Table 2.

Then, 100 parts by weight of phenyl thioglycidyl ether thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.63 and Abbe's number of 38. The results of observing the appearance showed that the lens was free from haze, but colored in pale yellow. The results are shown in Table 2.

### COMPARATIVE EXAMPLE 4

Into a flask equipped with a full zone stirring machine, a thermometer and an nitrogen inlet, were charged 530.0 g (2.5 mol) of 1,2,3-tris(β-epoxypropyl)propane, 761.2 g (10 mol) of thiourea, 41.0 g (0.4 mol) of acetic anhydride, 2.3 kg of toluene, and 2.1 kg of methanol. The mixture was allowed to react at 24°C for 12 h. After the reaction, 0.5 kg of water was added. The mixture was cooled to 15°C and stirred for 20 min. After the stirring was stopped, the separated aqueous layer was discarded. After further adding 0.5 kg of water, the mixture was stirred at 15°C for 20 min. After the stirring was stopped, the aqueous layer was discarded. Then, by removing toluene by distillation, 1,2,3-tris(β-epithiopropyl)propane was obtained. The nitrogen content is shown in Table 2.

Then, 100 parts by weight of 1,2,3-tris(β-epithiopropyl)propane thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.70 and Abbe's number of 37. The results of observing the appearance showed that the lens was clouded and colored in pale yellow. The results are shown in Table 2.

### COMPARATIVE EXAMPLE 5

The procedure of Comparative Example 1 was repeated except for using 2.5 mol of bis(β-epoxypropyl) disulfide in place of bis(β-epoxypropyl) sulfide to obtain bis(β-epithiopropyl) disulfide. The nitrogen content thereof is shown in Table 2.

Then, 100 parts by weight of bis(β-epithiopropyl) disulfide thus obtained was blended with 1.0 part by weight of triphenylphosphine, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.74 and Abbe's number of 34. The results of observing the appearance showed that the lens was free from haze, but colored in yellow. The results are shown in Table 2.

### COMPARATIVE EXAMPLE 6

The procedure of Comparative Example 1 was repeated except for using 2.5 mol of bis(β-epoxypropyl)selenide in place of bis(β-epoxypropyl) sulfide to obtain bis(β-epithiopropyl)selenide. The nitrogen content thereof is shown in Table 2.

Then, 100 parts by weight of bis(β-epithiopropyl)selenide thus obtained was blended with 0.1 part by weight of tetra-n-butylphosphonium bromide, and the mixture was injected into a 2-mm thick mold formed between two plates of glass. The polymerization was carried out by raising the temperature from 20°C to 90°C over 20 h to cure the mixture, thereby obtaining an optical material. The lens thus obtained has a refractive index of 1.73 and Abbe's number of 33. The results of observing the appearance showed that the lens was free from haze, but colored in yellow. The results are shown in Table 2.

**Table 2**

| Comparative Examples | Episulfide Compound | | Lens | |
|---|---|---|---|---|
| | Name | Nitrogen Content (ppm) | Haze | Yellowing (YI value) |
| 1 | bis(β-epithiopropyl)sulfide | 5524 | none | 3.98 |
| 2 | 1,2-bis(β-epithiopropylthio)ethane | 6239 | none | 4.27 |
| 3 | phenyl thioglycidyl ether | 5162 | none | 3.45 |
| 4 | 1,2,3-tris(β-epithiopropylthio)propane | 5424 | occurred | 3.75 |
| 5 | bis(β-epithiopropyl)disulfide | 7329 | occurred | 6.27 |
| 6 | bis(β-epithiopropyl)selenide | 6660 | occurred | 8.37 |

### Industrial Applicability

The method of the present invention enables to produce an episulfide compound having, in one molecule, one or more epithio structures of Formula 1, which has a nitrogen content of 5000 ppm or less. An optical material produced from such an episulfide compound has a high refractive index and a large Abbe's number, with extremely low coloring and haze as compared with known optical materials.

## Claims

1. An episulfide compound having, in one molecule, one or more epithio structures represented by the following Formula 1: 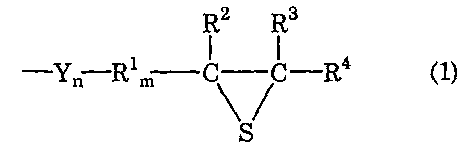 wherein R¹ is C₁-C₁₀ hydrocarbylene group; R², R³ and R⁴ are each independently C₁-C₁₀ hydrocarbyl group or hydrogen; Y is S, O, Se or Te; n is an integer of from 0 to 5; and m is 0 or 1,
the episulfide compound having a nitrogen content of 5000 ppm or less.

2. A method for producing an episulfide compound, comprising the steps of:
reacting thiourea with an epoxy compound having, in one molecule, one or more epoxy structures represented by the following Formula 2: 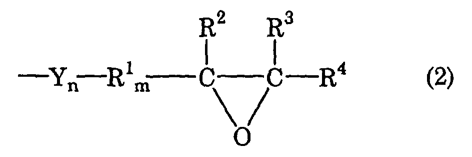 wherein R¹ is C₁-C₁₀ hydrocarbylene group; R², R³ and R⁴ are each independently C₁-C₁₀ hydrocarbyl group or hydrogen; Y is S, O, Se or Te; n is an integer of from 0 to 5; and m is 0 or 1,
in a mixed solvent of a water-soluble solvent and a water-insoluble solvent;
adding a water-soluble solvent and/or a water-insoluble solvent to the resultant reaction liquid;
mixing the resultant mixture and separating the mixture into an aqueous layer and a non-aqueous layer; and
isolating from the non-aqueous layer the episulfide compound as defined in Claim 1.

3. The method according to Claim 2, wherein the water-soluble solvent to be added to the reaction liquid is an aqueous solution of acid.

4. The method according to Claim 2, wherein the non-aqueous layer which is obtained by adding the water-soluble solvent and/or the water-insoluble solvent to the resultant reaction liquid, mixing the resultant mixture, and separating the mixture into an aqueous layer and a non-aqueous layer is repeatedly washed in a manner comprising the steps of:
adding water or an aqueous solution of acid to the non-aqueous layer;
separating the resultant mixture into the aqueous layer and the non-aqueous layer; and
removing the aqueous layer.

5. The method according to Claim 4, wherein the water-insoluble solvent is added in addition to water and the aqueous solution of acid.

6. The method according to any one of Claims 3 to 5, wherein the acid is at least one acid selected from the group consisting of hydrochloric acid, sulfuric acid, boric acid, phosphoric acid and acetic acid.

7. The method according to any one of Claims 3 to 6, wherein the acid concentration of the aqueous solution of acid is 0.1 to 90%.

8. An optical material produced by polymerizing the episulfide compound as defined in Claim 1 to a cured product.
